(19) Europäisches Patentamt
European Patent Office
Office européen des brevets

(11) EP 4 782 459 A1

(12) **EUROPEAN PATENT APPLICATION**

(43) Date of publication:
**29.07.2026 Bulletin 2026/31**

(21) Application number: **25460005.9**

(22) Date of filing: **24.01.2025**

(51) International Patent Classification (IPC):
***C07H 23/00*** *(2006.01)* ***C07H 1/00*** *(2006.01)*
***A61P 35/00*** *(2006.01)* ***B82Y 5/00*** *(2011.01)*
***B82Y 40/00*** *(2011.01)* ***C08F 2/10*** *(2006.01)*
***A61K 47/32*** *(2006.01)*

(52) Cooperative Patent Classification (CPC):
**C07H 1/00; A61K 47/6903; A61P 35/00; C07H 23/00;** B82Y 40/00

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
- **Uniwersytet Warszawski**
  **00-927 Warszawa (PL)**
- **Universität Zürich**
  **8006 Zürich (CH)**

(72) Inventors:
- **Dagdelen, Serife**
  **02-678 Warszawa (PL)**
- **Karbarz, Marcin**
  **20-690 Warszawa (PL)**
- **Zelder, Felix Hubertus**
  **8006 Zürich (CH)**
- **Mestizo, Paula Daniela**
  **Zürich (CH)**

(74) Representative: **JD&P Patent Attorneys Joanna Dargiewicz & Partners**
**ul. Mysliborska 93A/50**
**03-185 Warszawa (PL)**

(54) **VITAMIN B12 MODIFIED MONOMER, VITAMIN B12 MODIFIED NANOGELS, METHODS OF THEIR SYNTHESIS AND USE IN TARGETED DRUG DELIVERY**

(57) The present invention relates to Vitamin B12 monomer having the structure represented by the formula 4 or 3, a method of synthesis of Vitamin B12 monomer having the structure represented by the formula 4 or 3, a method of synthesis of nanogels modified with Vitamin B12 using precipitation polymerization, a use of the nanogel modified with Vitamin B12 in drug delivery systems, a pharmaceutical composition comprising nanogel modified with Vitamin B12 and active ingredient, and a pharmaceutical composition comprising nanogel modified with Vitamin B12 and active ingredient for use in the treatment of cancer.

EP 4 782 459 A1

## Description

### Field of the invention

The present invention relates to the vitamin $B_{12}$ ($VB_{12}$) modified monomer as a building block for implementation in polymers like polymeric nanogelnanogels, which can be used in targeted drug delivery applications, in particular for cancer treatment, the vitamin $B_{12}$ ($VB_{12}$) modified nanogelnanogels, the methods of their synthesis, and the use of the vitamin $B_{12}$ ($VB_{12}$) modified nanogels in targeted drug delivery applications, in particular for cancer treatment.

### Background of the invention

Cancer remains one of the most critical global health challenges of the 21st century. It is not only a leading cause of death but also a significant burden on healthcare systems worldwide.[1] According to the International Agency for Research on Cancer (IARC), over 19.3 million new cancer cases were reported in 2022, with projections suggesting this figure will exceed 30 million by 2040. The most common cancers include breast, lung, colon and rectum, and prostate cancers.[2] Despite advancements in early diagnosis and treatments, the mortality rate remains alarmingly high, particularly for aggressive and late-stage cancers. Cancer now ranks as the primary cause of death for individuals under the age of 70 in many regions of the world.[3] Today, one in five people is expected to face a cancer diagnosis in their lifetime, making prevention an urgent public health priority.[4] In addition to its health implications, the socioeconomic impact of cancer, including the loss of productivity and the emotional toll on patients and families, underscores the urgency of finding more effective solutions.[5]

The primary approaches for cancer treatment-surgery, chemotherapy, and radiation therapy-have undoubtedly saved millions of lives. However, each comes with significant limitations. Surgery is often invasive and can only address localized tumors, leaving systemic cancers untreated.[6] Radiation therapy, while effective, can damage surrounding healthy tissues and lead to severe side effects.[7] Chemotherapy, a cornerstone of cancer treatment, relies on cytotoxic agents that attack rapidly dividing cells but lacks selectivity.[8] [9] Consequently, healthy tissues such as bone marrow, the gastrointestinal tract, and hair follicles are often affected, leading to systemic toxicity and debilitating side effects, including anemia, nausea, and immune suppression.[10] Moreover, chemotherapy is frequently hindered by poor drug solubility, low bioavailability, and the development of drug resistance, contributing to high rates of recurrence and limited long-term efficacy.[11]

To address these issues, researchers are focusing on methods to deliver drugs directly to tumors using nano- and micro-sized drug carriers. Various drug carriers, such as liposomes,[12] polymeric micelles,[13] lipids,[14] and hydrogels, have been developed to improve the precision and effectiveness of chemotherapy.[15] Among these, hydrogels have garnered significant attention as promising platforms for drug delivery applications due to their unique physicochemical properties and versatility. Composed of water and hydrophilic polymers, hydrogels-specifically their nanoparticle forms known as nanogels-are particularly effective due to their high water content, porosity, protection of the encapsulated drugs against degradation in the bloodstream, efficient treatment with reduced systemic toxicity, improved drug solubility and compatibility with a range of drugs.[16,17,18,19] These properties make them ideal for carrying and releasing cancer-fighting agents directly where they are needed in the body. While nanogel technology has advanced, designing nanogels that achieve safe and controlled drug release remains a challenge. Much research has be devoted to creating specialized nanogels that respond to specific triggers within the tumor environment. These stimuli, whether from within the body (such as enzymes,[20,21] pH levels,[22] reducing environment (presence of glutathione, GSH),[23] adenosine-5'-triphosphate (ATP)[24] or oxygen) or external sources (like heat,[25] magnetism,[26] ultrasound[27], or light[28]), enable nanogels to target tumors more accurately, allowing for effective treatment with lower doses.

Although single-response nanogels have made an impact, there is a growing need for more complex, multifunctional nanogels. Recently, multistimuli-responsive nanogels have demonstrated superior results by releasing drugs more precisely at tumor sites, outperforming single-response systems.[29,30,31] However, the current generation of nanogels often lacks tumor selectivity, relying on passive targeting mechanisms such as the enhanced permeability and retention (EPR) effect.[32] These approaches are limited by the heterogeneous vascularization of tumors and insufficient targeting precision, leading to suboptimal therapeutic outcomes.[33,34] While promising, further comprehensive analysis of these innovative systems is still needed.

To increase the capacity of targeting delivery of anticancer drugs to tumors, researchers functionalized nanogels with targeting agents, e.g., antibodies or folate.[35,36,37] However, specific functional limitations of antibodies have been identified, including restricted penetration into solid tumor masses and limited activation or interaction with immune effector mechanisms.[38] In the case of folate-functionalized nanogels, folic acid targets the folate receptor, which is not as selectively overexpressed across all types of cancer cells. Moreover, folic acid can have limited solubility and potential toxicity at higher concentrations.[39]

All living cells require vitamins for their survival. One essential nutrient for cell proliferation is vitamin $B_{12}$, also

known as $VB_{12}$ or cobalamin (Cbl). Therefore, rapidly dividing tissue, such as in malignant tumors, has high $VB_{12}$ requirements.[40,41]Additionally, its related receptor (transcobalamin II receptor, CD320) is overexpressed in many cancer cells.[42,43] Transcobalamin (TC)-bound $VB_{12}$ enters cells through receptor-mediated endocytosis, a process that requires calcium-dependent complex formation between TC and its specific cell surface receptor, CD320.[44] CD320 belongs to the Low-Density Lipoprotein Receptor (LDLR) family, which encompasses various multi-ligand receptors involved in cholesterol transport and other essential biological functions.[45] Unlike other receptors in the LDLR family that bind a range of different ligands, CD320 demonstrates high specificity and affinity exclusively for TC. This specificity is evident in that other LDLR ligands, such as LDL and RAP, do not interfere with TC binding, and both the full-length CD320 receptor and its soluble ectodomain bind TC with strong affinity.[46,47] Notably, even haptocorrin (HC), another high-affinity $VB_{12}$-binding protein with significant sequence and structural similarities to TC, is not recognized by CD320 despite also being present in plasma. Additionally, $VB_{12}$ is a water-soluble and nontoxic compound. This makes $VB_{12}$ an attractive entity for in vivo tumor diagnostic and possible therapeutic applications. [48] $VB_{12}$ has been extensively studied for its role in drug delivery applications, due to its specific uptake mechanisms.[49] [50] While $VB_{12}$ offers unique advantages as a drug delivery vector, there are notable limitations. Its molecular structure restricts the size and type of drugs that can be conjugated without compromising receptor recognition or drug delivery efficiency.[46] Moreover, $VB_{12}$ typically relies on passive targeting via receptor-mediated endocytosis, which, while effective, is less precise than systems that integrate active targeting with tumor-specific triggers such as pH or redox gradients. These challenges underscore the need for multifunctional drug delivery systems that combine $VB_{12}$ targeting with additional tumor-specific mechanisms to enhance therapeutic efficacy. Thepphankulngarm et al. explored $VB_{12}$'s potential in their study, "Combining Vitamin $B_{12}$ and cisplatin-loaded porous silica nanoparticles via coordination: a facile approach to prepare a targeted drug delivery system."[51] In their approach, $VB_{12}$ was attached to silica nanoparticles through a non-covalent coordination bond involving the cobalt center of $VB_{12}$. While this method is straightforward and easy to execute ("facile"), the use of coordination bonds presents limitations. Such bonds are inherently weaker and more prone to dissociation, particularly in the presence of competing ions like chloride ions found in biological fluids. Furthermore, coordination bonds are sensitive to the biological environment, where reduction or exchange reactions involving the cobalt center can result in premature detachment of $VB_{12}$ from the nanocarrier.[52] Notably, the study did not include cytotoxicity experiments, leaving the biological relevance and therapeutic potential of the system unvalidated. Additionally, the study focused solely on cisplatin, limiting its application to tumors that respond to platinum-based therapies. In another research, Guo et al. explored $VB_{12}$'s potential in their study, " Vitamin-$B_{12}$-conjugated sericin micelles for targeting CD320-overexpressed gastric cancer and reversing drug resistance."[53] While this approach effectively combines $VB_{12}$ targeting and stimuli-responsive release, the use of sericin micelles presents certain limitations. The conjugation method primarily relies on non-covalent interactions which may lack the stability required for broader applications in dynamic physiological environments and targeting in long term will not be available. Carriers responsive only to pH which may not be enough for efficient drug release. Another significant limitation is non-degradable nature of micelles. They may persist in the body, particularly in the liver, spleen, or other organs, where nanoparticles are typically filtered. This can lead to long-term toxicity and undesirable side effects, especially for repeated or high-dose treatments.[54] Furthermore, the study focuses exclusively on gastric cancer, limiting the system's generalizability to other tumor types with varied microenvironments.

The object of the present invention was to provide a novel, more efficient and selective drug delivery system, which can be used particularly in the treatment of cancer.

## Summary of the invention

First subject of the present invention is Vitamin B12 monomer having the structure represented by the formula 4

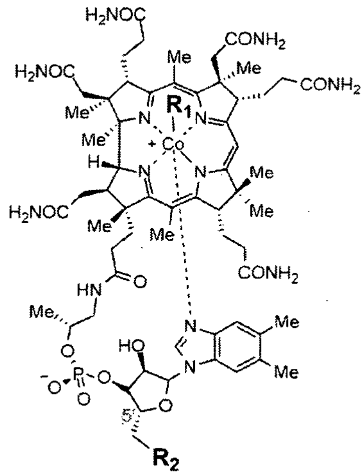

4

wherein:

$R_1$ is selected from the group $C_{15}H_{22}N_6O_5S$, $CH_3$, OH, CN, and

$R_2$ is selected from the group comprising carbamate, alkyl, aryl, amide, ester, thiol, or polymeric chains.

Preferably, Vitamin B12 monomer above defined has the structure represented by the formula 3

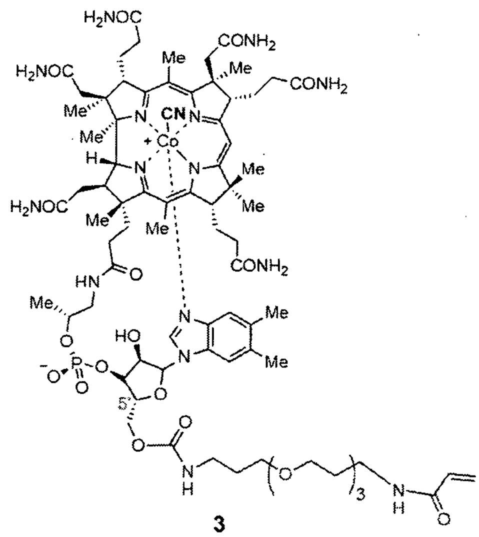

3

Second subject of the present invention is a method of synthesis of Vitamin B12 monomer having the structure represented by the formula 3 or 4 comprising

in step one

- activation of the Cyanocobalamin by activation of the 5'-hydroxyl group of the ribose moiety, wherein the activation is achieved through esterification, tosylation, halogenation, or similar reactions, and

in step two

- conjugation using versatile reactions such as carbamate formation, nucleophilic substitution, click chemistry, or amide bond formation.

Preferably, a method of synthesis of Vitamin B12 monomer having the structure represented by the formula 3

comprises

in step one

- activation of the Cyanocobalamin at the 5' hydroxide (-OH) ribose position using CDT;
- carbamate coupling reaction of the previously 5'-activated Cyanocobalamin with 4,7,10-trioxa-1,13-tridecadiamine and DIPEA with stirring and heating;
- precipitation of the reaction product and purification; and

in step two

- dissolution of the reaction product obtained in step one in DMF;
- reaction of the dissolved product with N,N-Diisopropylethylamine and acryloyl chloride at 0°C;
- precipitation of the reaction product and purification.

Preferably, the reaction is carried out in DMSO under gentle heating.
Preferably, acryloyl chloride is added 30 minutes after N,N-Diisopropylethylamine.
Third subject of the present invention is a method of synthesis of nanogels modified with Vitamin B12 using precipitation polymerization, comprising:

- dissolution of a main monomer NIPA, a comonomer AA and a linker BAC in deionized water,
- heating and deoxygenating of the reaction mixture;
- addition of APS and TEMED to initiate the reaction;
- addition of the Vitamin B12 monomer and stirring the reaction mixture;
- cooling the reaction mixture to the room temperature;
- precipitation of the reaction product and purification.

Preferably, the pH is adjusted to 5.0, and/or the reaction mixture is heated to 55°C, and/or the reaction mixture is deoxygenated for 0.5 h using argon.
Preferably, the Vitamin B12 monomer is selected from the monomers having the structure represented by the formula 3 or 4, or obtained by the method defined above.
Fourth subject of the present invention is use of the nanogel modified with Vitamin B12 in drug delivery systems.
Another subject of the present invention is nanogel modified with Vitamin B12 for use in the treatment.
Preferably, nanogel modified with Vitamin B12 is for use in the treatment of cancer.
Another subject of the present invention is a pharmaceutical composition comprising nanogel modified with Vitamin B12 and active ingredient.
Preferably, the active ingredient is anticancer drug, such as doxorubicine.
Preferably, the pharmaceutical composition comprising nanogel modified with Vitamin B12 and active ingredient is for use in the treatment of cancer.

## Brief description of the figures

So that the manner in which the above recited features, advantages and objects of the present disclosure are attained and can be understood in detail, a more particular description of this disclosure, briefly summarized above, may be had by reference to the embodiments thereof which are illustrated in the appended drawings.
It is to be noted, however, that the appended drawings illustrate only typical embodiments of this disclosure and are therefore not to be considered limiting of its scope, for this disclosure may admit to other equally effective embodiments.

FIG. 1 presents synthesis scheme of 5'-VB12 monomer (a) and p(5'-VB12-AA-BAC-NIPA) nanogels (b). Visual confirmation of incorporation of 5'-VB12 monomer in the polymer network and purification of nanogel via centrifugation and resuspension (c).

FIG. 2 presents chemical characterizations a) UV absorption spectra of p(AA-BAC-NIPA) nanogels, 5'-$VB_{12}$ monomer, ***p***(5'-$VB_{12}$-AA-BAC-NIPA) nanogels, ***p***(5'-$VB_{12}$-AA-BAC-NIPA)/DOX nanogels and DOX. b) FTIR spectra of 5'-$VB_{12}$ monomer, p(AA-BAC-NIPA) nanogels and ***p*** (5'-$VB_{12}$-AA-BAC-NIPA) nanogels.

FIG. 3 presents SEM and TEM images of the p(AA-BAC-NIPA) nanogels (column a) and p(5'-$VB_{12}$-AA-BAC-NIPA) nanogels (column b), SEM image and EDS elemental mapping of carbon (C), nitrogen (N), cobalt (Co) and combination of N and Co in the p(5'-$VB_{12}$-AA-BAC-NIPA) nanogels (column c).

FIG. 4 A comprehensive dynamic light scattering analysis illustrating (a) particle size distribution, (b) hydrodynamic diameter as a function of temperature, and (c) zeta potential as a function of pH for p(AA-BAC-NIPA), and p(5'-$VB_{12}$-AA-BAC-NIPA) nanogels (d) temperature-dependent hydrodynamic diameter variations at different pH levels for p(AA-BAC-NIPA), and for (e) p(5'-VB12-AA-BAC-NIPA) nanogels.

FIG. 5 presents a) Raman spectra of 5'-$VB_{12}$ monomer and p(5'-VB12-AA-BAC-NIPA) nanogels under different conditions. The spectra include the untreated nanogel, nanogel incubated with 10 mM GSH, and nanogel incubated with 40 mM GSH. b) Mean diameter distribution graphs for ***p***(5'-$VB_{12}$-AA-BAC-NIPA) nanogels with 10 uM, 10 mM, 40 mM GSH concentrations and treating time. c) TEM images of ***p*** (5'-$VB_{12}$-AA-BAC-NIPA) nanogel particles before degradation and degradation products obtained after 6 hours of interaction with 40 mM GSH (pH 5.0).

FIG. 6 presents release profiles of DOX alone and DOX from p(AA-BAC-NIPA) nanogels (A) and ***p***(5'-$VB_{12}$-AA-BAC-NIPA) nanogels (B) were examined in buffered solutions at 37°C. Glutathione was introduced at the concentrations specified in the graphs.

FIG. 7 presents hydrodynamic diameter changes plotted vs. time in HEPES solution at 4°C, 25°C and 37°C and mean diameter distribution changes in time, in fetal bovine serum at 37°C for ***p***(5'-$VB_{12}$-AA-BAC-NIPA) nanogels (A, B) and ***p*** (5'-$VB_{12}$-AA-BAC-NIPA)/DOX nanogels (C, D).

FIG. 8 presents the results of MTT assay using A549 (a), MRC-5 (b), HT29 (c) and CCD 841 CoN (d) cell lines after 72-h treatment with DOX free and DOX-loaded p(AA-BAC-NIPA) and ***p*** p(5'-VB12-AA-BAC-NIPA) nanogels.

FIG. 9 presents the confocal images of A549, HT-29, , MRC-5, and CCD-841 CoN treated with nanogels loaded with DOX and free DOX at $IC_{50}$ doses for 72h. CCD 841 CoN cells were exposed to $IC_{50}$ doses determined for HT-29. Images were captured in both bright field and fluorescence modes under 40× magnification to detect compound accumulation. Scale bar 20 μm.

FIG. 10 presents scheme of action of the nanogels according to the present invention loaded with doxorubicine.

**Detailed description**

The present invention relates to novel $VB_{12}$ derivatives-conjugated degradable nanogels which can be used as versatile drug delivery systems, combining multiresponsive properties with selective targeting capabilities. By leveraging $VB_{12}$-functionalization and stimuli-responsive mechanisms, these nanogels as drug carriers, in particular doxorubicin carriers, offer a promising platform for advanced cancer therapy, with broad applicability across various tumor environments and therapeutic agents.

The nanogels according to the present invention are designed to respond to multiple tumor-specific stimuli, including elevated levels of glutathione (GSH), acidic pH, and redox gradients within the tumor microenvironment. Among various types of cancer, lung cancer, laryngeal cancer, mouth cancer, and breast cancer exhibit higher concentrations (10-40 mM) of glutathione (GSH) compared to healthy cells.[55] As a result, drug delivery systems containing disulfide bonds, typically cross-linked micro-nanogels, are particularly effective due to their ability to degrade in the presence of high GSH concentrations.[56] This degradation process releases the drug payload specifically into cancerous or tumorous tissue, leveraging the significant difference in redox potential between the oxidizing extracellular environment and the reducing intracellular cytosol.[57,58] Additionally, the nanogel's temperature-sensitive properties enhance drug loading efficiency through reversible pore size changes, effectively trapping therapeutic agents.

A novel derivative of $VB_{12}$ was specifically designed and synthesized by targeting the 5'-position of the ribose moiety in cyanocobalamin. This position was selected for chemical modification due to its critical role in preserving the binding affinity to transcobalamin II (TCII) and ensuring efficient receptor-mediated delivery via the TCII receptor (TCII-R). TCll-mediated delivery is a key part of our approach, as many cancer cells, including those studied here, overexpress TCII-R (CD320), making this pathway an effective mechanism for selectively targeting cancer cells. The 5'-position provides a versatile and accessible site for functionalization without significantly disrupting the natural interaction between vitamin $B_{12}$ and TCII, enabling the targeted delivery of therapeutic agents to cancer cells.[59] The 5'-position of the ribose moiety represents as a suitable site for introducing a variable functional group $R_2$, where R encompasses a broad range of chemical functionalities. These may include, but are not limited to, carbamate, alkyl, aryl, amide, ester, thiol, or polymeric chains. This formula enables the design and protection of derivatives tailored for diverse therapeutic, diagnostic, or targeting applications.

Other potential modification sites in cyanocobalamin, such as the α-axial ligand, β-axial ligand, and the corrin ring, were not preferred for this application due to their limitations in preserving TCII binding and receptor-mediated targeting. Modifications at the α-axial ligand, while suitable for radiolabeling or metal-based drug delivery, can alter the electronic properties of the cobalt center, weakening its interaction with TCII and potentially reducing transport efficiency. Similarly, changes to the β-axial ligand can introduce steric hindrance that disrupts TCII-R recognition, compromising the selectivity and efficacy of receptor-mediated delivery. Alterations to the corrin ring or its amide side chains, though useful for modifying the molecule's solubility or pharmacokinetics, risk destabilizing the overall structure of cyanocobalamin and significantly impairing its ability to bind TCII.[60 61]

The synthesis of the novel derivative of $VB_{12}$ according to the present invention comprises modular steps, including activation of the 5'-hydroxyl group and conjugation via versatile coupling reactions, enabling the creation of diverse monomers while preserving TCII compatibility. The method for synthesizing such derivatives involves a modular approach focused on the selective activation of the 5'-hydroxyl group of the ribose moiety. This activation can be achieved through esterification, tosylation, halogenation, or similar strategies, followed by conjugation using versatile reactions such as nucleophilic substitution, click chemistry, or amide bond formation. These reactions allow the attachment of a variety of $R_2$ groups while preserving the biological compatibility and functionality of vitamin B12 derivatives for TCll-mediated delivery.

By integrating this new derivative of VB12 (5'-VB12) functionalization with multistimuli-responsive design, this invention offers dual functionalities: enhanced targeting through receptor-mediated uptake -provided by the 5'-VB12 monomer-, and precise drug release at tumor sites -provided by the proposed nanogels. This approach reduces off-target effects, improves therapeutic efficacy, and minimizes systemic toxicity, addressing the limitations of current nanogel technologies. Moreover, the versatility of this system allows for the encapsulation of a wide range of therapeutic agents, including both hydrophilic and hydrophobic drugs, broadening its applicability to various cancer types and treatment regimens. The invention's emphasis on creating degradable nanogels ensures environmental and biological safety, addressing concerns regarding the persistence of nanomaterials in the body and the environment. Additionally, the invention provides a scalable and cost-effective manufacturing process, making it feasible for widespread clinical application.

## EXAMPLES

### Materials

N-isopropylacrylamide (NIPA, 97%), ammonium persulfate (APS, 99.99%), acrylic acid (AA, 99%), *N,N'*-bis(acryloyl)cystamine (BAC), and L-glutathione reduced (GSH, 98%) were purchased from *Sigma-Aldrich.* Sodium hydroxide (NaOH, 99%), hydrochloric acid (HCl, 35-38%), *N,N,N',N'*-Tetramethyl ethylenediamine (TEMED) and were purchased from POCH. Doxorubicin hydrochloride (DOX) was purchased from *LC Laboratories (Woburn, MA,* USA). Fetal bovine serum (FBS) was purchased from *ScienCell Research Laboratories.* HEPES solution (4-(2-hydroxyethyl)-1-piperazineethanesulfonic acid, 1M, pH 7.0-7.6) was purchased from *Sigma-Aldrich (St. Louis, MO,* USA) and used without further purification. 4,7,10-Trioxa-1,13-tridecanediamine (97%) was obtained from Sigma-Aldrich and used as received without further purification. All chemicals were used in their original form as supplied by the manufacturers, except for NIPA, which was recrystallized twice from a benzene/hexane (9:1) mixture before use in the experiments. All solutions were prepared using high-purity water, which was obtained from a Hydrolab/HLP purification system, with a water conductivity of 0.056 $\mu S\ cm^{-1}$.

Chemicals were of reagent grade quality or better and obtained from *Sigma-Aldrich, ACROS Organics, Merck* or *Fluka* and used without further purification unless otherwise indicated. $B_{12}$ (1; CNCbl) was obtained from *Sigma-Aldrich.* All solvents were of reagent, analytical, HPLC or LC-MS grade, respectively. $H_2O$ from a *Milli-Q (Merck-Millipore)* water purification system was used for UV/Vis spectroscopy, mass spectrometry and when indicated. Reactions were monitored for completion by LC-MS. Evaporation of the solvents *in vacuo* was done with the rotary evaporator (*Büchi*) at the given

bath temperature and pressure. SepPak® *RP-18* cartridges (Waters) were applied for solid phase extraction (SPE). In general, the cartridges were conditioned by passing MeOH (5 mL) followed by $H_2O$ (20 mL). Afterwards, the compounds were dissolved in $H_2O$ or a buffer, transferred to the adsorbent, washed with $H_2O$, and eluted with $CH_3OH$. Variations from this protocol such as the use of buffer instead of $H_2O$ are indicated in the individual procedures. Every time a final product was obtained from precipitation after preparative HPLC purification, the solid was sedimented using a centrifuge 5702 *(Eppendorf)* at indicated speed and time and the solvent mixture decanted carefully. The final product centrifugation procedure was always performed in the vial where the solid was finally stored. All final products were dried under high vacuum (HV) overnight using a Schlenk line at a pressure not higher than $8 \cdot 10^{-2}$ mbar.

**Cytotoxicity Materials**

Dimethyl sulfoxide (DMSO, 99%) was purchased from POCH, Gliwice, Poland, McCoy's 5A Medium, Minimum Essential Medium Eagle (MEM), MTT (3-(4,5-Dimethyl-2-thiazolyl)-2,5-diphenyl-2H-tetrazolium bromide), Nutrient Mixture F-12 Ham, Penicillin-Streptomycin, and phosphate-buffered saline (PBS) were purchased from Merck/Sigma-Aldrich (St. Louis, MO, USA). Fetal bovine serum (FBS) and Trypsin-EDTA 10× were purchased from Biowest (Riverside, MO, USA).

**Instrumental**

*Preparative high pressure liquid chromatography (HPLC)*

The purification of the compounds was performed on a Nexera *prep* HPLC system *(Shimadzu USA Manufacturing,* Inc., USA) equipped with an SPD-20A prominex UV/Vis detector (set to 280 nm), a LC-20AP solvent delivery unit and a CBM-20A/20Alite system controller. The columns used for the separation (from *Macherey-Nagel*) were either a Nucleosil 100-7 C18 250/40 column, used at a 40 mL $min^{-1}$ flow; or 250/21 column, used at an 18 mL $min^{-1}$ flow. The solvent system employed is indicated in each individual method. The crude to be separated was first dried under high vacuum and then dissolved in the solvent mixture corresponding to the starting conditions of the method to be employed (injection loop volume = maximum 5.0 mL).

Method 1: An isocratic method (0 min 20% A, 0-32 min 20% A, 32-40 min 20-95% A) of $CH_3CN$ containing $CF_3COOH$ (0.1%; solvent A) vs. an aq. soln. of $CF_3COOH$ (0.1%; solvent B) was applied using a flow rate of 40 mL $min^{-1}$.

*Liquid chromatography-mass spectrometry (LC-MS)*

LC-MS was performed on an *Vanquish™ Horizon UHPLC* System *(Thermo Fisher Scientific,* Waltham, USA) connected to a *Vanquish™* eλ detector and ISQ-EM mass spectrometer using a Heated Electrospray ionization (HESI) source *(Thermo Fisher Scientific,* Waltham, USA), operated in positive or negative ESI mode at 3000 V (-2'000 V) capillary voltage with a $N_2$ sheath gas pressure of 41.9 psi, auxiliar gas pressure of 5.5 psi and sweep gas pressure of 0.1 psi. Vaporizer temperature was 338°C. Spectra were acquired in the mass range m/z 200-2000. Separation was performed with an Acquity UPLC BEH C18 column Gravity 1.7 μm (2.1 mm × 50 mm) reversed phase column *(Waters Corporation,* USA), kept at 40°C. The mobile phase consisted of A: $CH_3CN$ + 0.1% HCOOH and B: $H_2O$ + 0.1% HCOOH. UV spectra were recorded between 190 and 670 nm at a 4 nm resolution and at 5 Hz.. Samples were dissolved in $H_2O$ or $CH_3CN$ and a total volume of 2.0μL of the sample was analyzed. Method: A gradient (0 min 5.0% A, 0-0.5 min 5.0% A, 0.5-2.0 min 5-30% A, 2.01-4.0 min 30-100% A, 4.01-5.0 min 100% A) of $CH_3CN$ (solvent A) vs. an aq. soln. of 0.1% HCOOH (solvent B) was applied using a flow rate of 0.6 mL $min^{-1}$.

*High resolution electrospray ionization* mass *spectrometry (HR-ESI-MS)*

High resolution electrospray ionization mass spectrometry (HR-ESI-MS) was performed on a *Dionex Ultimate 3000* UHPLC system *(Thermo Fischer Scientific,* Germering, Germany) connected to a QExactive MS with a heated ESI source *(Thermo Fisher Scientific,* Bremen, Germany); onflow injection of 1 μL sample (c = ca. 50 μg $mL^{-1}$ in the indicated solvent) with an XRS auto-sampler *(CTC,* Zwingen, Switzerland); flow rate 120 μL $min^{-1}$; ESI: spray voltage 3 kV, capillary temperature 280°C, sheath gas 30 L $min^{-1}$, aux gas 8 L $min^{-1}$, s-lens RF level 55.0, aux gas temperature 250°C ($N_2$); full scan MS in the alternating (+)/(-)-ESI mode; mass ranges 80-1'200 m/z, 133-2'000 m/z, or 200-3'000 m/z at 70'000 resolution (full width half-maximum); automatic gain control target of $3 \times 10^6$; maximum allowed ion transfer time (IT) 30 ms; mass calibration to <2 ppm accuracy with *Pierce*® ESI calibration solns. *(Thermo Fisher Scientific,* Rockford, USA); lock masses: ubiquitous erucamide (m/z 338.34174, (+)-ESI) and palmitic acid (m/z 255.23295, (-)-ESI).

*Nuclear magnetic resonance spectroscopy (NMR)*

Both $^1$H- and $^{13}$C-NMR spectra were carried out at 298 K in DMSO-d6, $D_2O$ or $CD_3OD$ and at 500 MHz or 126 MHz, respectively. The $^1$H-NMR spectra were recorded with a *Bruker AVANCE NEO* 500 MHz spectrometer (Bruker, Germany) using a 5 mm-z-gradient RT-BBI probehead; $\delta$ in ppm relative to the residual signal of DMSO-d6 ($\delta$ 2.50; corresponds to TMS($\delta$ 0.00)), HDO ($\delta$ 4.79; corresponds to TMS ($\delta$ 0.00)) or $CHD_2OD$ ($\delta$ 3.31; corresponds to TMS ($\delta$ 0.00)), J in Hz. Spectra in $D_2O$ were presaturated. The $^{13}$C-NMR spectra were recorded with a *Bruker AVANCE NEO* 500 MHz spectrometer (Bruker, Germany) using a 5mm z-gradient CP-BBO probehead; $\delta$ in ppm relative to the residual signal of DMSO-d6 ($\delta$ 39.5; corresponds to TMS ($\delta$ 0.0)).

*Dynamic light scattering*

The hydrodynamic diameter of the particles was determined using a Malvern Zetasizer instrument (Nano ZS, UK). The instrument was equipped with a 4-mW Helium-Neon laser, which emitted light at a wavelength of 632.8 nm, and a scattering angle of 173° was used for the measurements. Before taking the measurements, the samples were allowed to equilibrate at the desired temperatures for 5 minutes. The changes in particle hydrodynamic diameter were then measured as a function of temperature.

*Fourier transform infrared (FTIR)*

The FTIR spectra were recorded using a Fourier Transform Infrared Spectrometer, Nicolet iS50 FT-IR spectrometer (Thermo Scientific) with DTGS detector. The instrument was equipped with a universal attenuated total reflectance (ATR) accessory, which allows for convenient and efficient sample analysis.

*Electron microscopy (SEM and TEM)*

SEM micrographs were obtained using a Merlin instrument manufactured by Zeiss. The instrument was operated at 3 kV. To prepare the samples, a drop of gel dispersion was placed on the SEM table and allowed to dry at room temperature. Prior to imaging, the samples were coated with a 5-nm layer of Au-Pd alloy using a Mini Sputter Coater (Polaron SC7620) under vacuum conditions. TEM micrographs were captured using a Zeiss Libra 1200 instrument. A drop of gel dispersion was placed on a formvar-coated copper grid, and the samples were left to dry at room temperature. Subsequently, the grid with the dried samples was used for TEM imaging. The average size of the microparticles was determined using Nano Measurer 1.2 software based on TEM images

*Raman spectroscopy*

Raman analyses were carried out using a Raman microscope (LabRAM Aramis, Horiba Jobin Yvon S.A.S, Lille, France) equipped with a 532 nm laser source at room temperature. For each measurement, lyophilized samples were prepared, and a few grains of the powder were placed on a CaF2 disk (Crystran LTD, Poole, UK).

Calibration was performed using the silicon band at 520.7 $cm^{-1}$. Raman spectra of each sample were recorded over a range of 100 to 3500 $cm^{-1}$ with a spectral resolution of 0.8 $cm^{-1}$. The spectra acquisition was managed using LabSpec6 software (version 6.4, Horiba Scientific SAS, Palaiseau, France).

*Confocal microscopy imaging*

Confocal Laser Scanning Microscopy (CLSM) was used to determine intracellular localization of tested compounds. All cells were seeded into a glass-bottom 24-well plate at a density of 2×104 cells/well for A549 and HT-29 cells, 5×104 cells/well for MRC-5, 7×103 cells/well for CCD 841 CoN,. After 24h of preincubation cells were treated with the IC50 values of tested compounds. After 72h of incubation, the imaging was performed using the Zeiss LSM 800 Axio Observer 7 inverted confocal microscope with a CCD camera, in both bright field and fluorescence modes (Zeiss, Oberkochen, Germany). Image acquisition was performed under 40× magnification and processed in ZEN Blue 2.6 software (Zeiss, Oberkochen, Germany).

***Other measurements***

The pH of the aqueous dispersion of nanogels was controlled by adding either sodium hydroxide or hydrochloride solution, and the pH was monitored using a Mettler Toledo model SevenGo-SG2 pH-meter just before the measurements. The ionic strength of the suspensions was maintained at a constant value of 0.01 M. In experiments involving the use of

GSH as a reducing agent, the pH of the suspensions was adjusted to approximately 5.0.

**Example 1**

*Synthesis of 5'-$VB_{12}$* monomer

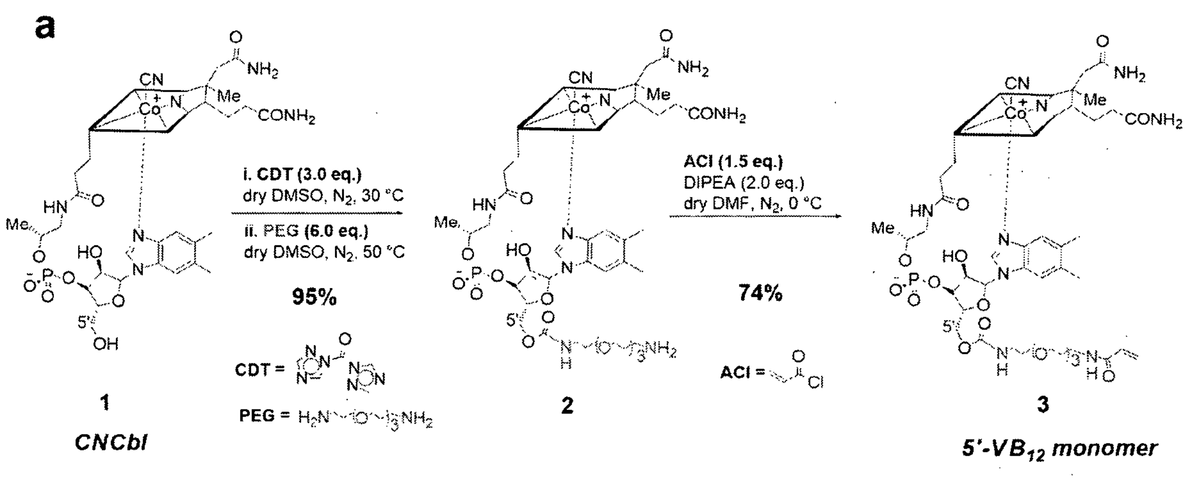


**Scheme 1a.** Synthesis scheme of 5'-$VB_{12}$ monomer (a).

5'-$VB_{12}$ monomer was synthesized in two steps starting from commercially available $B_{12}$ (Cyanocobalamin, CNCbl,) **1** in a total isolated yield of 70.3%. The synthesis of **2** (Scheme S1) was slightly modified according to a reported procedure.[62]

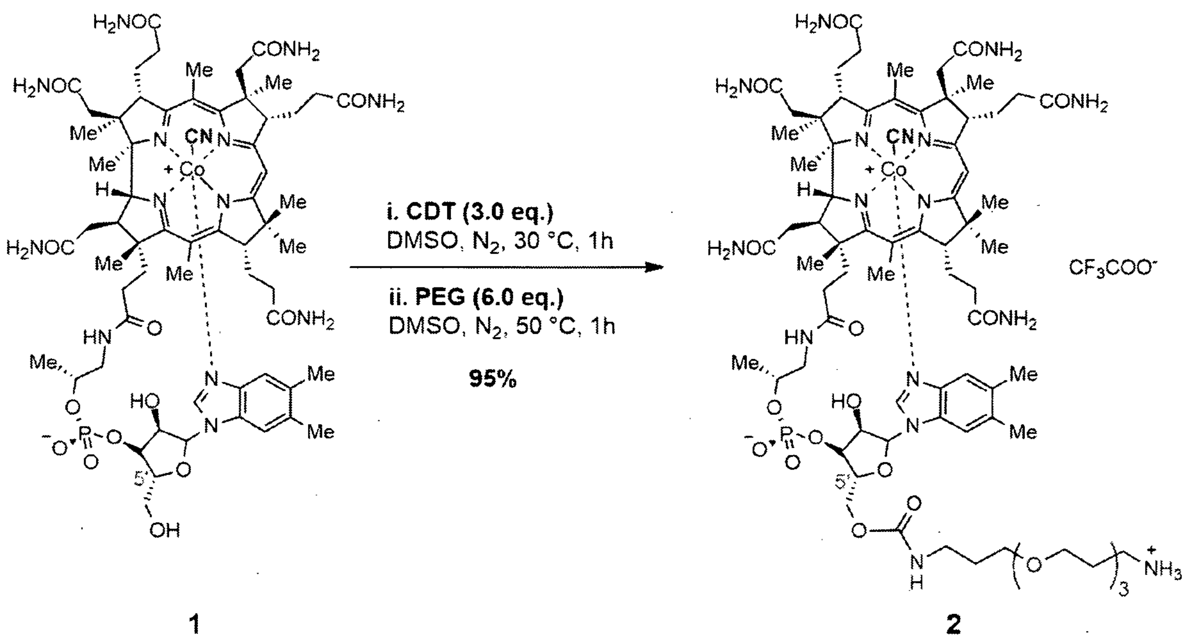


**1**

$C_{63}H_{88}CoN_{14}O_{14}P$
1355.39 g/mol

**2**

$C_{76}H_{111}CoF_3N_{16}O_{20}P$
1715.72 g/mol

***Scheme S1.*** *Synthesis of CNCbl-5'-$PEG_3$ (**2**) from Vitamin $B_{12}$ (**1**).*

In a Schlenk flask and under an inert $N_{2(g)}$ atmosphere, CNCbl (200.0 mg, 0.148 mmol, 1.0 eq.) was added to DMSO (1.5 mL) under stirring. After the solid was completely dissolved, CDT (N,N'-Carbonyl-di-(1,2,4-triazole)) (72.8 mg, 0.442 mmol, 3.0 eq.) was added. The reaction mixture was then heated to 30°C for 1h. The activation step was completed

after 1h as indicated by reaction control using LC-MS. After, 4,7,10-trioxa-1,13-tridecadiamine (192 μL, 0.88 mmol, 6 eq.) was added. The reaction mixture was left stirring for an additional 1h under the same temperature. The resulting crude mixture was precipitated over $Et_2O$: DCM 2:1 (10 mL), centrifuged and the DMSO rich supernatant discarded. The remaining crude was washed using DCM (2 mL x 2) and $Et_2O$ (5 mL x 2). After drying the solid to remove all organic solvents, it was purified using preparative HPLC (method 1, $t_{ret}$= 11.2 min). $CH_3CN$ was then evaporated from the collected pure fractions and the product passed through a SepPack C18 cartridge, washed with $H_2O$ (20 mL) and finally eluted with MeOH (5mL). The final red solid was obtained after precipitation from a concentrated MeOH solution (200 μL final volume) using methyl tert-butyl ether (MTBE) (166.9 mg, 0.097 mmol, 95 %).

**UV-Vis** ($H_2O$, c = $4.0 \cdot 10^{-5}$ M): (4.25), 519 (3.71), 549 (3.75). **LC-MS** (MS+) ($t_{ret}$ = 1.73 min): 801.36 (100, [M - $CF_3COO$ + H]$^{2+}$, m/$z_{calc}$: 801.37), 1601.85 (20, [M - $CF_3COO$]$^+$, m/$z_{calc}$: 1601.73 for $C_{74}H_{111}CoN_{16}O_{18}P^+$).

**$^1$H NMR** (DMSO-d6, c = $1.2 \cdot 10^{-2}$ M) δ 8.07 (s, 3H), 7.71 (d, *J* = 9.5 Hz, 2H), 7.64 (s, 1H), 7.56 (d, *J* = 16.0 Hz, 2H), 7.37 (s, 1H), 7.28 (s, 1H), 7.19 (s, 2H), 7.16 (s, 1H), 7.08 (s, 1H), 7.03 (s, 1H), 6.99 (s, 1H), 6.79 (s, 1H), 6.72 (s, 1H), 6.53 (s, 1H), 6.44 (s, 1H), 6.41 (s, 1H), 6.20 (s, 1H), 5.92 (s, 1H), 4.68 (d, *J* = 7.2 Hz, 1H), 4.63 (q, *J* = 7.7 Hz, 1H), 4.25 (d, *J* = 10.5 Hz, 1H), 4.20 - 4.11 (m, 2H), 4.09 (d, *J* = 7.4 Hz, 1H), 4.04 (s, 1H), 3.94 (d, *J* = 11.1 Hz, 1H), 3.75 - 3.67 (m, 1H), 3.58 - 3.45 (m, 12H), 3.45 - 3.38 (m, 2H), 2.99 - 2.90 (m, 1H), 2.84 (dd, *J* = 12.3, 6.4 Hz, 2H), 2.80 - 2.74 (m, 1H), 2.58 (d, *J* = 12.2 Hz, 1H), 2.48 (s, 3H), 2.44 (s, 2H), 2.38 (s, 3H), 2.37 - 2.30 (m, 3H), 2.26 (t, *J* = 14.6 Hz, 2H), 2.16 (d, *J* = 7.2 Hz, 8H), 2.08 - 1.96 (m, 3H), 1.79 (q, *J* = 7.1, 6.1 Hz, 5H), 1.73 (s, 2H), 1.69 (s, 3H), 1.63 (dt, *J* = 14.7, 6.9 Hz, 3H), 1.51 (dt, *J* = 15.1, 8.0 Hz, 1H), 1.32 (s, 3H), 1.23 (s, 3H), 1.18 (s, 3H), 1.07 (d,*J* = 7.2 Hz, 7H), 0.87 (s, 1H), 0.33 (s, 3H).

**$^{13}$C NMR** (DMSO-d6, c = $1.2 \cdot 10^{-2}$ M) δ 179.88, 178.48, 175.52, 174.49, 173.96, 173.94, 173.50, 173.04, 172.98, 171.74, 171.71, 165.60, 165.06, 156.58, 142.43, 136.60, 133.27, 131.87, 129.82, 116.67, 116.27, 111.52, 106.35, 103.34, 93.93, 86.33, 84.66, 79.67, 75.01, 73.11, 72.56, 71.52, 70.04, 69.96, 69.76, 69.74, 68.93, 68.04, 67.78, 63.08, 58.93, 55.34, 54.31, 53.35, 50.70, 49.06, 47.71, 46.89, 44.80, 42.36, 42.12, 38.39, 37.88, 37.21, 35.36, 34.17, 32.02, 31.94, 31.77, 31.66, 30.99, 30.58, 29.80, 27.47, 26.12, 25.91, 20.23, 20.20, 20.12, 20.09, 19.96, 19.00, 16.75, 16.71, 15.90, 15.30, 1.41.

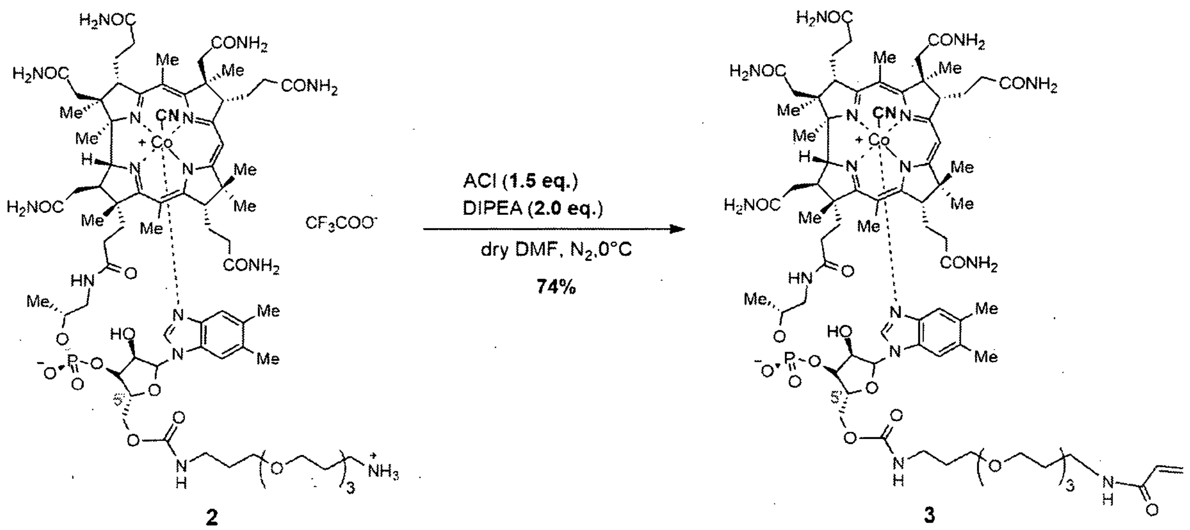


2

$C_{76}H_{111}CoF_3N_{16}O_{20}P$
1715.72 g/mol

3

$C_{77}H_{112}CoN_{16}O_{19}P$
1655.72 g/mol

***Scheme S2.** Synthesis of CNCbl-5'-PEG$_3$-ACl (**3**) from CNCbl-5'-PEG$_3$ (**2**).*

In the second step (Scheme S2), in a Schlenk flask and under an inert $N_{2(g)}$ atmosphere, CNCbl-5'-$PEG_3$ (compound 2) (100.0 mg, 0.062 mmol, 1.0 eq.) was added to DMF (2 mL) under stirring. After the solid was completely dissolved, *N,N*-Diisopropylethylamine (DIPEA) (19.3 μL, 0.12 mmol, 2.0 eq.) was added. The reaction mixture was then cooled to 0°C for 30 mins. After, acryloyl chloride (ACl) (7.6 μL, 0.094 mmol, 1.5 eq.) was added at 0°C. The reaction was completed after 1 h as indicated by reaction control using LC-MS. The resulting crude mixture was precipitated over $Et_2O$: DCM 2:1 (15 mL), centrifuged and the DMF rich supernatant discarded. The remaining crude was washed using DCM (2 mL x 2) and $Et_2O$ (5 mL x 2). After drying the solid to remove all organic solvents, it was purified using preparative HPLC (method 1, $t_{ret}$= 11.2 min). $CH_3CN$ was then evaporated from the collected pure fractions and the product passed through a SepPack C18 cartridge, washed with $H_2O$ (20 mL) and finally eluted with MeOH (5mL). The final red solid was obtained after precipitation from a concentrated MeOH solution (200 μL final volume) using methyl tert-butyl ether (MTBE) (166.9 mg, 0.097 mmol,74 %).

**UV-Vis** ($H_2O$, c = 4.0·$10^{-5}$ M): 280.4, 361.7, 519, 549.

**LC-MS** (MS+) ($t_{ret}$ = 1.99 min): 828.53 (100, *[M* + 2H]$^{2+}$, m/$z_{calc}$: 828.37), 1655.82 (20, [M + H]$^{+}$, m/$z_{calc}$: 1655.74 for $C_{77}H_{112}CoN_{16}O_{19}P$).

**$^{1}$H NMR** (DMSO-d6, c = 1.1.$10^{-2}$M) δ 8.19 (t, *J* = 5.3 Hz, 1H), 7.77 (s, 1H), 7.72 (d, *J* = 19.0 Hz, 2H), 7.57 (d, *J* = 36.3 Hz, 2H), 7.36 (d, *J* = 7.4 Hz, 3H), 7.17 (d, *J* = 26.1 Hz, 2H), 7.08 (s, 1H), 7.01 (d, *J* = 13.0 Hz, 2H), 6.76 (d, *J* = 19.1 Hz, 2H), 6.53 (s, 1H), 6.43 (s, 1H), 6.37 (s, 1H), 6.28 - 6.17 (m, 2H), 6.05 (dd, *J* = 17.1, 2.2 Hz, 1H), 5.89 (s, 1H), 5.54 (dd, *J* = 10.2, 2.2 Hz, 1H), 4.67 (d, *J* = 7.4 Hz, 1H), 4.63 (s, 1H), 4.29 (d, *J* = 10.4 Hz, 1H), 4.19 - 4.11 (m, 1H), 4.08 (d, *J* = 7.6 Hz, 1H), 4.02 (dd, *J* = 11.2, 7.2 Hz, 2H), 3.93 (d, *J* = 10.8 Hz, 1H), 3.69 (dd, *J* = 9.9, 5.9 Hz, 1H), 3.51 (dt, *J* = 5.4, 3.1 Hz, 3H), 3.49 - 3.43 (m, 4H), 3.42 - 3.35 (m, 1H), 3.19 - 3.12 (m, 2H), 3.02 (q, *J* = 6.8, 6.3 Hz, 2H), 2.88 (dd, *J* = 13.9, 7.0 Hz, 1H), 2.81 - 2.73 (m, 1H), 2.58 (d, *J* = 12.2 Hz, 1H), 2.46 (d, *J* = 9.2 Hz, 4H), 2.40 - 2.34 (m, 5H), 2.17 (d, *J* = 7.7 Hz, 6H), 2.02 (dd, *J* = 20.0, 8.8 Hz, 3H), 1.86 - 1.73 (m, 4H), 1.73 - 1.56 (m, 11H), 1.50 (dt, *J* = 14.4, 7.3 Hz, 2H), 1.32 (s, 3H), 1.23 (s, 4H), 1.18 (s, 3H), 1.04 (d, *J* = 9.5 Hz, 6H), 0.91 - 0.80 (m, 1H), 0.32 (s, 3H).

**$^{13}$C NMR** (DMSO-d6, c = 1.1.$10^{-2}$M) δ 179.53, 178.25, 175.13, 173.97, 173.67, 173.25, 172.81, 172.53, 171.13, 170.98, 165.39, 164.66, 164.59, 156.16, 142.23, 136.25, 132.66, 131.91, 131.28, 129.77, 124.79, 120.36, 116.38, 111.42, 105.85, 103.08, 93.57, 85.94, 84.38, 79.48, 74.77, 72.92, 72.10, 70.40, 69.78, 69.55, 68.83, 68.09, 67.95, 63.14, 58.63, 54.95, 54.01, 52.97, 50.33, 48.76, 47.37, 46.55, 44.57, 42.09, 41.85, 40.20, 38.07, 37.59, 35.90, 35.13, 33.99, 31.66, 31.05, 29.89, 29.63, 29.27, 27.16, 25.79, 25.56, 20.09, 20.04, 19.93, 19.89, 19.84, 18.72, 16.51, 16.40, 15.65, 15.05.

The structures of the monomers and the method of synthesis of the 5'-$VB_{12}$ monomer are presented in Schemes 1a, S1 and S2.

**Example 3**

Synthesis *of 5' positioned Vitamin $B_{12}$ (5'-$VB_{12}$) monomer modified nanogels*

**a**

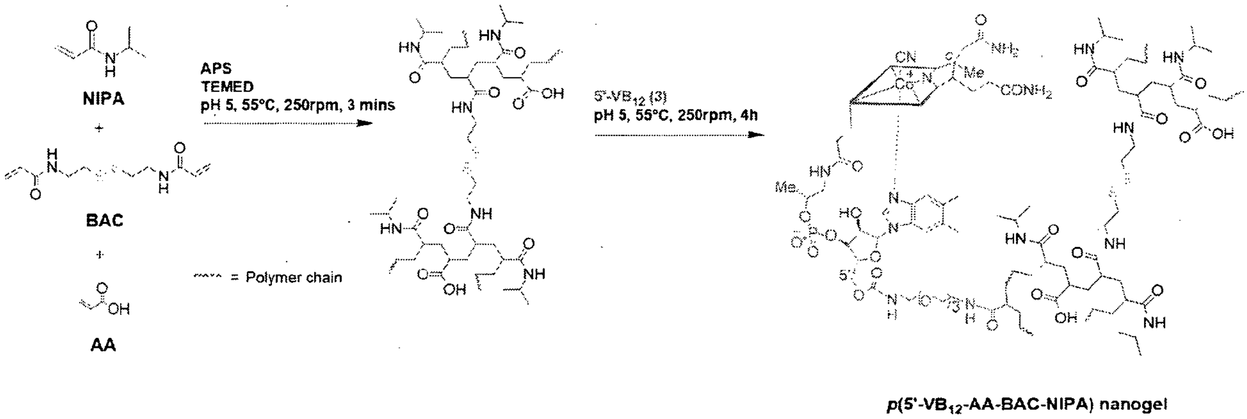


***Scheme 1b.*** Synthesis scheme of *p*(5'-$VB_{12}$-AA-BAC-NIPA) nanogels (b).

The p(5'-VB12-AA-BAC-NIPA) nanogels were synthesized using precipitation polymerization. Polymerization was conducted using a three-neck flask equipped with a reflux condenser, inlet, and outlet of inert gas. In the first step, the main monomer NIPA (0.1901 g, 84 mM), comonomer AA (0.0137 mL, 10 mM) and linker BAC (0.0052 g, 1 mM, in 0.5 mL EtOH) were dissolved in 15 mL of deionized water. pH of mixture changed to 5.0 to ensure the stability of 5'-$VB_{12}$ during the polymerization process and placed in the flask. The mixture was heated to 55°C and deoxygenated for 0.5 h using argon to maintaining the reaction's efficiency. After mechanical stirring (250 rpm) for 1 h, an aqueous solution of APS (0.009 g in 1 mL deionized water) and 7 μL of TEMED, as a accelerator, were added to initiate the reaction. After few minutes of initiating of the reaction, 5'-$VB_{12}$ (0.1656 g, 5 mM, in 4 mL deionized water) was immediately added to the mixture. The reaction was allowed to proceed for 4 h, stopped by cooling the product to room temperature. The structures of the monomers and the method of synthesis of the 5'-$VB_{12}$-nanogels are presented in Scheme 1b. The product was purified via dialysis (dialysis tube with a 10,000 Da molecular weight cutoff-Spectra/Por® 7 Dialysis Membrane) against deionized water (pH 3.0 for 2 h, followed by pH 7.0 for 24 h). This cycle was repeated three times to remove unreacted monomers. The purified nanogel solution, free of residual 5'-$VB_{12}$, was confirmed by the absence of 5'-$VB_{12}$'s characteristic absorbance (361 nm) in the

supernatant. Additionally, after purification procedure, to verify the absence of unbound 5'-$VB_{12}$ in the nanogel suspension, the solution was subjected to centrifugation (Hermle Z 32 HK) at 20,000 × g for 10 minutes at room temperature. After centrifugation, the supernatant was inspected visually and spectroscopically for any residual 5'-$VB_{12}$, characterized by its distinct color and absorbance profile (see Scheme 1c).

Similarly, p(AA-BAC-NIPA) nanogels without 5'-$VB_{12}$ were synthesized as controls to evaluate the efficiency of 5'-$VB_{12}$-modified systems. The concentration of these nanogels in solution, expressed as their dried mass, was approximately 5 mg/mL.

**Example 5**

*Loading and release of drug*

Loading of doxorubicin (an anti-cancer drug) was carried out by cooling (25°C) and heating (42°C) a mixture of nanogel and drug. For this purpose, 3 mL of p(5'-VB12-AA-BAC-NIPA) nanogel was mixed with 3 mL of HEPES buffer (pH 7.4; 0.1M) containing 1.5 mg DOX. The solution was then heated to 42°C for 10 min and left at 25°C for 24h. The resulting solution was placed into dialysis bags with a 10 kilodalton molecular weight cutoff (MWCO) and place in HEPES buffer to remove excess of non-bounded DOX.

Release of the drug was performed using dialysis bags (10 kDa MWCO) and was done by putting 1 mL of pure ***þ*** (5'-$VB_{12}$-AA-BAC-NIPA) + DOX nanogel (1.7 mg gel and 0.17 mg DOX) in the dialysis bags and placed bags in 9 mL of HEPES or acetate buffer (pH 7.4 and 5.0; 0.1M) containing different concentration of GSH. The release was conducted at 37°C with 100 rpm magnetic stirring. The absorbance of the buffers at 480 nm was measured to calculate mass of released doxorubicin.

The loading and release procedure was performed in the same manner for the p(AA-BAC-NIPA) nanogel.

**Cell examination**

*Cell Lines and Culture Conditions*

In the presented study, four cell lines purchased from the American Type Culture Collection ( ATCC; Manassas, VA, USA) were used: two carcinomas, A549, HT-29, and two normal, MRC-5, CCD 841 CoN,. Human non-small-cell lung carcinoma A549 were cultured in Nutrient Mixture F-12 Ham medium, colon adenocarcinoma HT-29 cells were cultured in McCoy's 5A medium. Media were supplemented with 10% heat-inactivated fetal bovine serum, 100 μg/mL of streptomycin, and 100 U/mL of penicillin. Human lung fibroblast MRC-5 cells and colon epithelial CCD 841 CoN cells were maintained in Minimum Essential Medium Eagle supplemented with 10% FBS without antibiotics. The cells were incubated at 37 °C in a 5% $CO_2$ humidified atmosphere. Experiments were performed with the cells in the exponential phase of growth.

*Cytotoxicity assay*

The cytotoxicity of the tested compounds was determined using the 3-(4,5-dimethylthiazol-2-yl)-2,5 diphenyltetrazolium bromide (MTT). All cells were seeded into a 96-well plate at a density of 2×103 cells/well for A549 and HT-29 cells, 5×103 cells/well for MRC-5, 7×103 cells/well for CCD 841 CoN. After 24h of preincubation cells were treated with the tested compound at concentrations up to 60 μM prepared in sterile water. After 72h of treatment, MTT (400 ug/well) was added for 2h, then the formazan crystals were dissolved in DMSO, and absorbance was measured using a microplate reader (iMarkTM, Bio-Rad, USA) at 540 nm. The compound concentrations that inhibited cell growth by 50% ($IC_{50}$) compared with the untreated, control cells were calculated from curves plotting survival as a function of dose. Results were obtained from at least four independent experiments.

**Results and discussion**

The chemical structural characterization of 5'-$VB_{12}$ monomer, p(AA-BAC-NIPA) nanogels and p(5'-VB12 -AA-BAC-NIPA) nanogel was performed using UV-Vis and FTIR. Results are presented in FIG. 2.

The UV-Vis absorption spectra presented in FIG. 2.a indicate the presence of 5'-VB12 in the nanogel. The spectrum for the p(5'-VB12-AA-BAC-NIPA) nanogel displays a prominent absorption peak at 362 nm, which is characteristic of the corrin ring in VB12. In contrast, the baseline spectrum for the unloaded p(AA-BAC-NIPA) nanogel reveals no significant absorption features, highlighting the specific contributions associated with the presence of 5'-VB12. Moreover, the absence of color in the supernatant confirms both the incorporation of 5'-VB12 into the nanogel and the cleanliness of the suspension. The pellet could be easily resuspended, demonstrating the homogeneity and stability of

the nanogel system (see Fig. 1c). However, the UV-Vis data alone does not differentiate between the physical entrapment and the covalent bonding of the 5'-VB12 monomer to the polymer network of the nanogel. Further characterization was performed using FTIR spectroscopy, which provided more detailed insights into the nature of the incorporation of the 5'-$VB_{12}$ monomer.

The FTIR spectra in FIG. 2.b also confirm the presence of 5'-$VB_{12}$ into the nanogel through distinct functional group signatures. For the 5'-$VB_{12}$ monomer, key peaks include the C≡N stretch at 2138 $cm^{-1}$, indicating the presence of the cyanide group, and the C=O stretch at 1667 $cm^{-1}$, attributed to the propionamide side chain. [63,64]Additionally, peaks at 3327 $cm^{-1}$ and 3202 $cm^{-1}$ correspond to O-H and N-H stretching, respectively, confirming the hydroxyl and amide groups in the 5'-$VB_{12}$ structure.[65] For the ***p*** (5'-$VB_{12}$-AA-BAC-NIPA) nanogel, the spectrum shows a slight shift of the C≡N peak to 2134 $cm^{-1}$, indicating successful incorporation of 5'-$VB_{12}$ monomer into the nanogel. The C=O stretching peak at 1647 $cm^{-1}$ represents the combined contribution of both the amide groups in the polymer and the 5'-$VB_{12}$ monomer structure, further verifying the integration. Peaks between 1540 $cm^{-1}$ and 1457 $cm^{-1}$ correspond to N-H bending and C-H bending from both the polymer and the corrin ring in 5'-$VB_{12}$.[63,52] Moreover, the P-O stretch at 1085 $cm^{-1}$, characteristic of phosphate groups and C-O-C stretching vibrations at 999 $cm^{-1}$ reflect the presence of ether linkages in 5'-$VB_{12}$ monomer, further supports its structural integration.[66,67] In comparison, the p(AA-BAC-NIPA) nanogel spectrum shows no C≡N, P-O and C-O-C peaks confirming the absence of 5'-$VB_{12}$ monomer. Instead, it exhibits typical peaks such as the strong C=O stretch at 1625 $cm^{-1}$ from the amide groups and the N-H bending at 1550 $cm^{-1}$, which are indicative of the N-isopropylacrylamide in the nanogel structure. [64,63] However, the absence of a signal near 1625 $cm^{-1}$ in the spectrum of the p(S'-$VB_{12}$-AA-BAC-NIPA) nanogel, which is typically associated with the vibration of the -C=C-bond in 5'-$VB_{12}$, clearly indicates that 5'-$VB_{12}$ is chemically incorporated into the nanogel polymer network during free radical polymerization. These results clearly differentiate the 5'-$VB_{12}$-loaded nanogel from the $VB_{12}$-free version, emphasizing the successful chemical functionalization of the former, making it a promising candidate for responsive drug delivery systems.

The morphology of the ***p*** (5'-$VB_{12}$-AA-BAC-NIPA) nanogels were examined by electron microscopies. Scanning electron microscopy (SEM), transmission electron microscopy (TEM) and Energy-dispersive X-ray spectroscopy (EDS) were used for this purpose (see FIG. 3).

The top image in column a shows a SEM image of the p(AA-BAC-NIPA) nanogels. The image reveals a uniform distribution of spherical particles. The bottom image in column a is a TEM image of the same nanogels, providing an illustration of separated nanogel particles. The nanogels exhibit a consistent morphology, and the internal structure of the particles is uniform. The mean sizes of the particles measured from the SEM and TEM were similar, at approximately 50 nm.

The top image in column b shows the SEM image of the p(5'-$VB_{12}$-AA-BAC-NIPA) nanogels. Similar to the p(AA-BAC-NIPA) nanogels, the particles appear spherical and uniformly distributed. However, the surface texture of these nanogels seems slightly rougher compared to the p(AA-BAC-NIPA) nanogels, which could be attributed to the incorporation of the 5'-$VB_{12}$ monomer.

The bottom image in column b is the TEM image of the p(5'-$VB_{12}$-AA-BAC-NIPA) nanogels. This image provides a more detailed view of the internal structure. The particles are distinct, with a consistent size distribution. The mean sizes of the particles measured from the SEM and TEM were similar, at approximately 60 nm.

The first image in column c is a TEM image that further illustrates the morphology of the p(5'-$VB_{12}$-AA-BAC-NIPA) nanogels. The uniform distribution and consistent morphology are reaffirmed in this image, with a focus on the elemental composition provided by EDS mapping. The subsequent images in column c represent EDS elemental mapping for carbon (C), nitrogen (N), and cobalt (Co). The C, N, and Co maps show a rather uniform distribution across the nanogels, consistent with the polymer backbone's presence throughout the material. Moreover, the figure in the last column presents maps of N and Co. Based on this image, it can be seen that Co, which is distinctive for 5'-$VB_{12}$, shows a uniform but more localized distribution on the outer part of the particle, reinforcing the successful integration of 5'-$VB_{12}$ into the nanogels and its localization.

FIG. 3 presents SEM and TEM images of the p(AA-BAC-NIPA) nanogels (column a) and p(5'-VB12-AA-BAC-NIPA) nanogels (column b), SEM image and EDS elemental mapping of carbon (C), nitrogen (N), cobalt (Co) and combination of N and Co in the p(5'-$VB_{12}$-AA-BAC-NIPA) nanogels (column c).

Next, the particle size distribution, temperature sensitivity, and pH sensitivity of p(AA-BAC-NIPA) and p(5'-VB12-AA-BAC-NIPA) nanogels in aqueous solutions were investigated using the DLS technique. The obtained results are presented in FIG. 4. The size distribution of the nanogels after purification at 25°C is presented in FIG. 4a. The pH of the solutions was approximately 6. The average size of p(AA-BAC-NIPA) nanogels was approximately 120 nm, while for p(5'-$VB_{12}$-AA-BAC-NIPA), it was 140 nm. The intensity percentage indicates that the majority of particles are within this narrow size range, suggesting a consistent and uniform size distribution across the different formulations. Nanogels containing VB12 derivatives demonstrate an approximate increase of 20 nm in particle size and 0.12 in the polydispersity index (PDI) compared to $VB_{12}$-free nanogels (see Table 1). This increase is likely attributable to the incorporation of $VB_{12}$ into the nanogel matrix. FIG. 4b demonstrates the temperature sensitivity of the nanogels, assessed through their Z-average diameter (d.nm). As the temperature increases from 25°C to 70°C, nanogels exhibit a decrease in particle size, consistent

with the typical behavior of thermoresponsive polymers such as NIPA.[68,69] Notably, the p(AA-BAC-NIPA) nanogels show a sharp reduction in Z-average diameter around 38°C, aligning with the lower critical solution temperature (LCST) of NIPA. For $VB_{12}$-modified nanogels, this transition occurs at approximately 44°C, beyond which the nanogels undergo collapse due to the dehydration of the polymer network. The p(AA-BAC-NIPA) nanogel demonstrates the most substantial size reduction, whereas the $VB_{12}$-modified nanogels exhibit a less pronounced shrinkage and a shift in LCST. This behavior suggests that the incorporation of $VB_{12}$ moieties alters the thermoresponsive properties of the nanogels. VB12 contains multiple functional groups that can form hydrogen bonds with water molecules. This might stabilize the hydrated state of the nanogels and delay the dehydration process, thus increasing the LCST.[70] FIG. 4c assesses the zeta potential of the nanogels as a function of pH. Zeta potential is a measure of the surface charge of particles and is crucial for understanding the stability and interaction potential of colloidal systems.[71] Both nanogels show a decrease in zeta potential as the pH increases from 2 to 9, moving from slightly positive to more negative values. This behavior is strongly linked to the pKa of poly(acrylic acid), approximately 4.5. At pH 4.5, there is a rapid decrease in zeta potential, reflecting the deprotonation of carboxylic groups. This shift causes a significant increase in surface negative charge. The p(5'-$VB_{12}$-AA-BAC-NIPA) nanogels show similar zeta potential behavior, indicating that the $VB_{12}$ modification does not significantly alter the overall charge behavior compared to the unmodified p(AA-BAC-NIPA) nanogel (see Table 1). The effect of pH on the size and swelling behavior of nanogels was assessed using DLS at various temperatures and three specific pH levels: 2.4, 5.3, and 11.8 (FIGs. 4d and 4e). It can be observed that the hydrodynamic diameter of the nanogels decreases with an increase in temperature. However, both temperature and pH significantly influence the swelling behavior of the nanogels. At pH 2.4, the acrylic acid groups are mostly protonated, reducing electrostatic repulsion and leading to aggregation above 30°C. In contrast, at pH values above the pKa of carboxylic groups, the nanogels swell due to the ionization of carboxyl groups, resulting in increased electrostatic repulsion and osmotic pressure. For example, p(AA-BAC-NIPA) nanogels were approximately 90 nm at 37°C and pH 5.3, but increased to around 115 nm at pH 11.8 due to enhanced swelling. However, at pH 5.3 and 50°C, the size decreased to 50 nm, emphasizing the predominant effect of temperature-induced collapse. Similarly, p(5'-$VB_{12}$-AA-BAC-N IPA) nanogels exhibited pH-dependent swelling behavior, with a size increase from 125 nm at pH 5.3 to 150 nm at pH 11.8 at 37°C, suggesting that the presence of VB12 slightly reduces pH sensitivity. Nonetheless, a decrease to 60 nm at pH 5.3 and 70°C confirmed that temperature and pH jointly affect nanogel size.

FIG. 4 presents a comprehensive dynamic light scattering analysis illustrating (a) particle size distribution (25°C, pH = 6), (b) hydrodynamic diameter as a function of temperature, and (c) zeta potential as a function of pH for p(AA-BAC-NIPA) and *þ* (5'-$VB_{12}$-AA-BAC-NIPA) nanogels, as well as temperature-dependent hydrodynamic diameter variations at different pH levels (2.4, 5.3, and 11.8) for (d) p(AA-BAC-NIPA) and (e) *þ* (5'-$VB_{12}$-AA-BAC-NIPA) nanogels.

The difference in particle sizes observed between SEM/TEM and DLS measurements is primarily attributable to the state of the nanogels during analysis. DLS measurements reflect the sizes of nanogels in aqueous solutions, which are influenced by swelling and hydration depending on temperature and pH. As such, DLS yields hydrodynamic diameters that include the solvent layer, resulting in larger size readings. In contrast, SEM/TEM imaging captures the actual physical size of the dried, anhydrous micropolymer particles, leading to smaller observed dimensions.[72,73]

**Table 1.** Size distributions and surface potentials of nanogels.

| Sample | Diameter (nm) | Polydispersity Index (PDI) | Zeta Potential (mV) |
|---|---|---|---|
| *þ* (AA-BAC-NIPA) | 119 ± 1.0 | 0.11 ± 0.01 | -17.0 ± 0.8 |
| *þ* (5'-$VB_{12}$-AA-BAC-NIPA) | 138 ± 1.4 | 0.23 ± 0.01 | -18.5 ± 0.6 |

Raman spectroscopy, DLS, and TEM were employed to quantitatively evaluate the degradation of *þ* (5'-$VB_{12}$-AA-BAC-NIPA) nanogels (FIG. 5). In this context, GSH, a potent reducing agent, donates electrons to the disulfide bonds (-S-S-) within the nanogels, initiating a thiol-disulfide exchange reaction. This reaction results in the cleavage of disulfide bonds, converting them into two thiol groups (-SH), thereby facilitating targeted drug release precisely where it is needed. As the disulfide bonds break, the nanogel degrades into smaller fragments. This degradation process can lead to/enhance the release of encapsulated drugs. The combination of Raman spectroscopy, DLS, and TEM allows for a comprehensive understanding of this degradation process and its implications for drug releasing.[74]

Panel (a) shows the Raman spectra of *þ* (5'-$VB_{12}$-AA-BAC-NIPA) nanogels exposed to different concentrations of GSH, highlighting the reductive degradation process through the cleavage of disulfide bonds (-S-S-). The spectra reveal distinct features that correspond to chemical changes in the nanogel structure. In the untreated nanogel, strong peaks associated with disulfide bonds are evident. Upon exposure to 10 mM GSH, the appearance of a new peak corresponding to thiol groups (SH) indicates partial reduction of disulfides. This effect is further intensified with 40 mM GSH, where the SS bonds are almost completely reduced, as demonstrated by a significant increase in SH signal and the near absence of SS peaks. These results confirm that higher concentrations of GSH lead to more pronounced nanogel degradation, with thiol

formation serving as a marker for the cleavage of disulfide linkages in the polymer network. FIG. 4.b presents the nanogel mean-size distribution measured after 0, 2, 12 and 24 hours of the treatment with various concentrations of GSH. The concentration of GSH in the cytoplasm is significantly higher (ranging from 0.5-10 mM) compared to extracellular fluids (2-20 μM), reaching levels up to 1000 times greater.[75 76]Tumor cells present higher levels of cytosolic GSH with respect to normal cells as a mechanism to promote cell survival and resistance to chemotherapy.[77] Among various types of cancer, lung cancer, larynx cancer, mouth cancer, and breast cancer exhibit higher concentrations (10-40 mM) of GSH compared to healthy cells.[78] To simulate the conditions around healthy and tumor cells, we used GSH concentrations of 10 μM, 10 mM, and 40 mM. As it can be seen in the graph, initially, the nanogels exhibited no significant change in diameter for up to 24 h after treatment with 10 μM GSH. Nanogel particles mean size increased from 121 ± 13 nm to 154 ± 34 nm. PDI value is increased from 0.22 to 0.24. After treatment with 10 mM GSH, nanogels were swollen, with a large size distribution evident in the graph, likely because of the cleavage of some disulfide bridges and loosening of the polymer network. After 24 hours of interaction, the nanogel particles mean size increased from 122 ± 42 nm to 372 ± 45 nm. PDI value is increased from 0.24 to 0.50. After treatment with 40 mM GSH, just after two hours, the nanogels started giving more than one peak in the size distribution graph. Further interactions of the nanogel with the reducing agent led to the cleavage of the rest of the -S-S- bridges; more peaks in the graph, corresponding to lower size distribution, were observed. However, DLS measurements become unreliable due to extensive degradation beyond 6 hours for 40 mM GSH treatment. This rapid increase indicates that higher GSH concentrations accelerate degradation, leading to fragmentation and possibly causing aggregation of degraded fragments, hence why the size measurements become difficult. We performed additional experimental measurements using TEM beyond the 6-hour mark to validate the extrapolated behavior. The transition of the morphology of the nanogels before and after degradation was observed by TEM (FIG. 5). The prepared sample was exposed to 40 mM GSH for 24 hours, followed by TEM analysis. After chemical reduction, no nanogel could be observed anymore, only polymer fragments resulting from the degradation step were present. This result indicates that the expected degradation was achieved successfully. Detailed redox-responsive behavior of p(5'-$VB_{12}$-AA-BAC-NIPA) nanogels are presented in Table 2.

**Table** 2.

Redox-responsive behavior of ƥ (5'-$VB_{12}$-AA-BAC-NIPA) nanogel.

| Time (h) | | 10 μM GSH | | 10 mM GSH | | 40 mM GSH | |
|---|---|---|---|---|---|---|---|
| | | Diameter (nm) | PDI | Diameter (nm) | PDI | Diameter (nm) | PDI |
| **ƥ (5'-$VB_{12}$-AA-BAC-NIPA) Nanogels** | 0 | 121 ± 13 | 0.22 | 122 ± 42 | 0.24 | 125 ± 37 | 0.29 |
| | 2 | 133 ± 43 | 0.21 | 205 ± 58 | 0.43 | 277 ± 69 | 0.48 |
| | 4 | 136 ± 45 | 0.23 | 254 ± 31 | 0.45 | 347 ± 86 | 0.60 |
| | 6 | 139 ± 50 | 0.21 | 271 ± 59 | 0.49 | 456 ± 52 | 0.61 |
| | 12 | 152 ± 42 | 0.21 | 310 ± 88 | 0.49 | - | - |
| | 24 | 154 ± 34 | 0.24 | 357 ± 73 | 0.50 | - | - |
| | 36 | 155 ± 51 | 0.24 | 372 ± 45 | 0.50 | - | - |

FIG. 5 presents a) Raman spectra of 5'-$VB_{12}$ monomer and p(5'-VB12-AA-BAC-NIPA) nanogels under different conditions. The spectra include the untreated nanogel, nanogel incubated with 10 mM GSH, and nanogel incubated with 40 mM GSH. b) Mean diameter distribution graphs for ƥ (5'-$VB_{12}$-AA-BAC-NIPA) nanogels with 10 uM, 10 mM, 40 mM GSH concentrations and treating time c) TEM images of ƥ (5'-$VB_{12}$-AA-BAC-NIPA) nanogel particles before degradation and degradation products obtained after 6 hours of interaction with 40 mM GSH (pH 5.0).

Next, the usefulness of the p(AA-BAC-NIPA) and ƥ (5'-$VB_{12}$-AA-BAC-NIPA) nanogels as doxorubicin carriers were examined. Loading of doxorubicin was conducted at pH 7.4. At this pH, doxorubicin is electrostatically bound to the polymeric network of nanogels; the ionized carboxylic groups of the polymer attract the protonated amine groups of doxorubicin. The heating and cooling process used during loading enhances drug loading efficiency by inducing reversible size changes in temperature-sensitive nanogels. For instance, at 25°C, the ƥ (5'-$VB_{12}$-AA-BAC-NIPA) nanogel has a size of 150 nm, but when the temperature is raised to 42°C, its size reduces to 70 nm. Consequently, the pores of the nanogel shrink, effectively trapping the drug within. The UV-Vis absorption spectra confirmed the successful loading of DOX into the nanogels. The spectra for the ƥ (5'-$VB_{12}$-AA-BAC-NIPA) nanogel show a distinct absorption peak around 480-500 nm, characteristic of doxorubicin together with peak at 362 nm characteristic for 5'-$VB_{12}$ (See FIG. 2a). Together, these results confirm that the nanogel system can stably incorporate both 5'-$VB_{12}$ for targeting and DOX for therapeutic action, making it a promising candidate for dual-functional drug delivery applications. The drug loading capacity (DLC) was assessed using

the formula: $DLC\% = \frac{m_{total}^{DOX} - m_{free}^{DOX}}{m_{nanogel\,DOX}} \times 100\%$, and it was equaled 10.9% and 10.4 % for p(AA-BAC-NIPA) and *p* (5'-$VB_{12}$-AA-BAC-NIPA) nanogels, respectively. The drug encapsulation efficiency (DEE) was assessed using the following equation: $DEE\% = \frac{m_{total}^{DOX} - m_{free}^{DOX}}{m_{total}^{DOX}} \times 100\%$, and it was equaled 78.2% and 69.8 % for p(AA-BAC-NIPA) and *p* (5'-$VB_{12}$-AA-BAC-NIPA) nanogels, respectively.

FIG. 6 shows DOX release profiles performed at 37°C in acetate and HEPES buffer (pH 5.0 and 7.4; 0.1 M) with various concentration of glutathione for p(AA-BAC-NIPA) and *p* (5'-$VB_{12}$-AA-BAC-NIPA) nanogels. It can be seen that at physiological pH, lower DOX release was observed, for both nanogel case because at this pH most carboxylic groups in nanogel are ionized and electrostatically bound to positively charged drug. While at pH 5.0, near to pKa of carboxylic groups (pKa ≈ 4.5) protonation of carboxylic groups in nanogels caused higher release. The addition of GSH caused an increase in DOX release from the nanogels due to their degradation and competitive interaction with ionized carboxylic groups. However, these effects depend on the concentration of GSH. The highest drug release was observed at condition typical for some cancer cells, it is low pH and presence of 40 mM GSH. In such condition, 56.3% and 74.4% of drug was released from p(AA-BAC-NIPA) nanogels, 57% and 74.4% of drug was released from *p* (5'-$VB_{12}$-AA-BAC-NIPA) nanogels after 4 and 83 h respectively and releasing was still increasing. Importantly in buffer pH 7.4 containing 10 μM GSH (extracellular fluids GSH level ranging 2-20 μM) and 10 mM (cytoplasm GSH level ranging from 0.5-10 mM) drug released after 83 h was 21.4% and 31.2% from p(AA-BAC-NIPA) nanogels, 24% and 33% from *p* (5'-$VB_{12}$-AA-BAC-NIPA) nanogels respectively. It can be concluded that the presence of GSH enhanced the release of the drug, and the shape of the release profiles differed significantly compared to cases without GSH. Notably, after the initial burst release that occurs in the first few hours, a sustained release is observed. This behavior can be attributed to the degradation of the nanogel carriers by GSH.

For comparative purposes, release experiments were conducted with free DOX, which demonstrated a very rapid total release under both pH conditions. In contrast, the nanogel exhibited a significantly slower and sustained release of DOX, highlighting its potential to retain the drug within the body for an extended period-an essential characteristic for effective therapeutic applications.

FIG. 6 presents release profiles of DOX alone and DOX from p(AA-BAC-NIPA) nanogels (A) and *p* (5'-$VB_{12}$-AA-BAC-NIPA) nanogels (B) were examined in buffered solutions at 37°C. Glutathione was introduced at the concentrations specified in the graphs.

The stability of p(5'-VB12-AA-BAC-NIPA) nanogels and *p* (5'-$VB_{12}$-AA-BAC-NIPA)/DOX nanogels in HEPES at 4°C, 25°C, and 37°C, and in fetal bovine serum (FBS) at 37°C, was evaluated using dynamic light scattering (DLS) (FIG. 7). The interaction with serum proteins is critical as it could affect the nanogels' behavior in vivo, including their circulation time, biodistribution, and cellular uptake. Understanding these interactions is crucial for predicting the nanogels' performance in biological environments. The temperatures correspond to storage conditions (4°C), drug loading and transport (25°C), and physiological conditions (37°C). Nanogels were diluted 100-fold in HEPES and 100% FBS to prevent serum saturation.

FIG. 7A shows, *p* (5'-$VB_{12}$-AA-BAC-NIPA) nanogels displayed stable hydrodynamic diameters in HEPES across all temperatures over 20 days. At 4°C, the size increased slightly from 122 nm to 128 nm, at 25°C from 115 nm to 120 nm, and at 37°C from 107 nm to 113 nm. The polydispersity index (PDI) showed minimal changes, increasing from 0.21 to 0.25, 0.28, and 0.31 at 4°C, 25°C, and 37°C, respectively, indicating good stability without significant aggregation.

FIG. 7B shows, in FBS at 37°C, p(5'-VB12-AA-BAC-NIPA) nanogels maintained a narrow size distribution over 72 hours. The mean diameter increased by only 10 nm within the first 2 hours and by another 22 nm between 2 and 12 hours, with the PDI increasing slightly to 0.32. No further significant changes were observed from 12 to 72 hours, suggesting minimal protein interaction and no major aggregation, maintaining structural stability throughout the exposure.

FIG. 7C shows, *p* (5'-$VB_{12}$-AA-BAC-NIPA)/DOX nanogels were stable in HEPES, showing size changes primarily within the first 5 days, after which they stabilized. The diameter increased from 131 nm to 154 nm at 4°C, from 120 nm to 150 nm at 25°C, and from 109 nm to 140 nm at 37°C over 20 days. The PDI rose slightly from 0.23 to 0.26, 0.31, and 0.36 at 4°C, 25°C, and 37°C, respectively, indicating stable dispersion with moderate temperature influence.

FIG. 7D shows, in FBS at 37°C, the DOX-loaded nanogels exhibited stable size distribution over 72 hours. The diameter increased by 21 nm within the first 2 hours and by an additional 23 nm between 2 and 12 hours, with the PDI increasing from 0.21 to 0.38. The nanogels maintained well-defined particles with no significant aggregation, suggesting compatibility with serum conditions and stable interaction dynamics.

FIG. 7 presents hydrodynamic diameter changes plotted vs. time in HEPES solution at 4°C, 25°C and 37°C and mean diameter distribution changes in time, in fetal bovine serum at 37°C for *p* (5'-VB12-AA-BAC-NIPA) nanogels (A, B) and *p* (5'-$VB_{12}$-AA-BAC-NIPA)/DOX nanogels (C, D).

The data reveals that both unloaded and DOX-loaded *p* (5'-$VB_{12}$-AA-BAC-NIPA) nanogels exhibit stable

hydrodynamic diameters in HEPES buffer at varying temperatures, with minimal size fluctuations over time. The results indicate that both unloaded and DOX-loaded *p* (5'-$VB_{12}$-AA-BAC-NIPA) nanogels maintain good stability in fetal bovine serum (FBS) at 37°C, with only moderate increases in size over time. The observed shifts in size distribution suggest some level of interaction with serum proteins, but these interactions do not lead to significant aggregation or destabilization of the nanogels. This stability in FBS is promising, as it suggests that the nanogels retain their structural integrity and functional properties in a complex biological environment, making them suitable candidates for drug delivery applications with predictable behavior in vivo.

## CYTOTOXICITY

The antiproliferative activity of nanogels and their conjugates with DOX was evaluated using the MTT assay on two cancerous cell lines, A549 and HT-29, and their corresponding normal cell lines, MRC-5 and CCD 841 CoN. The obtained results are presented in FIG. 8.

Generally, two trends are visible. In the case of cancer cells, $VB_{12}$-conjugated nanogel, p(5'-$VB_{12}$-AA-BAC-NIPA), consistently exhibited superior performance compared to the non-conjugated nanogel, p(AA-BAC-NIPA), demonstrating enhanced targeting capability and selectivity. This trend is better highlighted for HT-29 cells, where p(5'-$VB_{12}$-AA-BAC-NIPA) nanogels demonstrated a substantial improvement in efficacy, with an $IC_{50}$ (the concentration of a drug required to inhibit cell proliferation by 50%) of 0.878 $\pm$ 0.102 $\mu$M compared to 1.751 $\pm$ 0.418 $\mu$M for the p(AA-BAC-NIPA) nanogels. For A549 lung adenocarcinoma cells, the $IC_{50}$ for p(5'-$VB_{12}$-AA-BAC-NIPA) nanogels was 1.602 $\pm$ 0.147 $\mu$M, which was lower than the $IC_{50}$ for p(AA-BAC-NIPA) nanogels (1.801 $\pm$ 0.244 $\mu$M), indicating slightly improved cytotoxicity with $VB_{12}$-conjugation. However, for both A549 and HT-29 cells, free DOX demonstrated much higher cytotoxicity, with $IC_{50}$ values of 0.396 $\pm$ 0.117 $\mu$M and 0.222 $\pm$ 0.064 $\mu$M, respectively.

The second trend is related to the protective effect on normal cells, indicating that the cytotoxicity of DOX in nanogel carriers is significantly lower toward normal cells. In the case of MRC-5 cells, the $IC_{50}$ values were 3.048 $\pm$ 0.521 $\mu$M and 3.309 $\pm$ 0.801 $\mu$M for p(5'-$VB_{12}$-AA-BAC-NIPA) nanogels and p(AA-BAC-NIPA) nanogels, respectively. In addition, the cytotoxicity of DOX in nanogels is much lower for normal cells than DOX alone (0.687 $\pm$ 0.102 $\mu$M), reflecting reduced off-target toxicity. For CCD 841 CoN normal colon epithelial cells, the protective effect is even more significant, with $IC_{50}$ values of approximately 30 $\mu$M and 20 $\mu$M for p(5'-$VB_{12}$-AA-BAC-NIPA) nanogels and p(5'-VB12-AA-BAC-NIPA) nanogels, respectively. Free DOX exhibited an $IC_{50}$ of 3.681 $\mu$M, further confirming the protective effects of nanogel-DOX conjugates.

Both nanogels, p(5'-VB12-AA-BAC-NIPA) and p(AA-BAC-NIPA), devoid of DOX demonstrated no observable cytotoxic effects in all examined cell lines across the range of concentrations tested.

These findings underscore the critical role of $VB_{12}$ conjugation in enhancing the selectivity and efficacy of the nanogels. The p(5'-VB12-AA-BAC-NIPA) nanogels consistently showed lower IC50 values compared to the p(AA-BAC-NIPA) nanogels in cancer cells, while also exhibiting significantly reduced cytotoxicity in normal cells when compared to free DOX. The data highlights $VB_{12}$'s contribution to improving targeted delivery, making $VB_{12}$-conjugated nanogels a promising platform for safer and more effective cancer therapy.

FIG. 8 presents the results of MTT assay using A549 (a), MRC-5 (b), HT29 (c) and CCD 841 CoN (d) cell lines after 72-h treatment with DOX-loaded p(AA-BAC-NIPA) and *p* (5'-$VB_{12}$-AA-BAC-NIPA) nanogels.

## Compound accumulation / Cell imaging

The fluorescent properties of doxorubicin allowed us for the microscopic determination of compound accumulation in the cells of all tested lines (FIG. 9). Confocal microscopy imaging revealed similar patterns of doxorubicin accumulation in cancerous and normal cells treated with free doxorubicin and nanogels loaded with DOX. Tested compounds accumulation was predominantly observed in the cytoplasm and around the nucleus. The A549, and HT-29 cancer cells exposed to conjugates and DOX alone exhibited altered morphology. Shrinkage of cells with an increase in compound accumulation compared to cells with unchanged morphology indicates induction of cell death. In contrast, normal lung, MRC-5, and colon, CCD 841 CoN cells, showed mildly altered morphology after exposure to the compounds compared to controls, despite the observation of compound accumulation in the cytoplasm. However, considering the high $IC_{50}$ doses of these compounds against MRC-5 cells and CCD 841 CoN cells, the observed accumulation indicates that the key to the activity of the conjugates is not penetration into the cytoplasm, but penetration into the nucleus, where doxorubicin by DNA intercalation causes a toxic effect.

FIG. 9 presents the confocal images of A549, HT-29, MCF7, MRC-5, and CCD-841 CoN treated with nanogels loaded with DOX and free DOX at $IC_{50}$ doses for 72h. CCD 841 CoN cells were exposed to $IC_{50}$ doses determined for HT-29. Images were captured in both bright field and fluorescence modes under 40$\times$ magnification to detect compound accumulation. Scale bar 20 $\mu$m.

**Conclusions**

The 5'-$VB_{12}$-functionalized degradable nanogels according to the present invention offer a novel and transformative approach to targeted drug delivery, distinguished by several key differentiators that set them apart from existing solutions. Unlike conventional systems relying on non-covalent interactions, which are prone to instability in physiological environments, the present nanogels utilize covalent bonding for 5'-$VB_{12}$ attachment, ensuring exceptional stability and preventing premature detachment of the targeting ligand. This stability is further complemented by the multistimuli-responsive design of the nanogels, which respond to tumor-specific triggers such as acidic pH (thanks to carboxylic groups present in the monomer, acrylic acid), elevated glutathione (GSH) levels (thanks to -S-S- bridges present in the crosslinker, BAC), and redox gradients, enabling highly precise and controlled drug release within the tumor microenvironment. 5'-modified $VB_{12}$, as a functional ligand, specifically targets the transcobalamin II receptor (CD320), which is overexpressed in many cancer cells (breast, lung, colorectal, gastric, prostate, ovarian cancers)", offering superior tumor selectivity compared to traditional targeting ligands like antibodies, which suffer from poor tumor penetration, or folate, which is limited by variable receptor expression across cancer types. Additionally, the 5'-$VB_{12}$-modified nanogels according to the present invention exhibit higher water solubility and non-toxicity compared to folate-decorated nanogels. Moreover, $VB_{12}$ is naturally water-soluble and non-toxic, which reduces the risk of adverse effects. In contrast, folic acid can have limited solubility and potential toxicity at higher concentrations.[39] This increased safety profile makes our technology a more attractive option for cancer therapy. Additionally, the nanogel matrix is biodegradable, breaking down into components that can be safely excreted, addressing a critical limitation of non-degradable carriers such as micelles or inorganic nanoparticles, which risk accumulation in the liver and spleen and long-term toxicity. Furthermore, the present nanogels are highly versatile, capable of encapsulating both hydrophilic and hydrophobic drugs, making them adaptable to various therapeutic agents and cancer types. This versatility, combined with the scalable and cost-effective manufacturing process, ensures feasibility for clinical translation. Importantly, this platform is the first of its kind to integrate 5'-$VB_{12}$ functionalization with multistimuli-responsive degradable nanogels, addressing the limitations of existing drug delivery systems and establishing a groundbreaking solution that enhances therapeutic efficacy, safety, and applicability across diverse cancer therapies.

<u>Detail the mechanism of action and uniqueness of technology (FIG. 10)</u>

The proposed technology integrates 5'-$VB_{12}$-functionalized, multistimuli-responsive degradable nanogels as a novel platform for precise and effective drug delivery in cancer therapy. The mechanism of action is rooted in the specific targeting of the transcobalamin II receptor (CD320), which is overexpressed in various cancers, including breast, gastric, colorectal, and ovarian cancers. 5'-$VB_{12}$, covalently conjugated to the nanogel matrix, enables receptor-mediated endocytosis, ensuring selective uptake by tumor cells and minimizing off-target effects. This covalent attachment is a pivotal advancement over conventional systems relying on weaker non-covalent interactions, which are prone to dissociation under physiological conditions. The nanogels are engineered to respond to multiple tumor-specific stimuli, including acidic pH, a hallmark of the tumor microenvironment, and elevated glutathione (GSH) levels, characteristic of the intracellular milieu of cancer cells. Anticancer drug, doxorubicin, is electrostatically bound to the polymeric network of nanogels; the ionized carboxylic groups of the polymers attract the protonated amine groups of doxorubicin at pH 7.4. At acidic pH levels (pH 5.0), the nanogel matrix destabilizes, initiating controlled drug release while it stays stable and do not let the drug release at neutral pH (7.4). Simultaneously, the high GSH concentrations (10-40 mM) in cancer cells versus ~2-10 $\mu$M in healthy cells) cleave disulfide crosslinks within the nanogel, enabling precise intracellular drug delivery while at low concentration of GSH (10 $\mu$M) nanogels were quite stable. This dual responsiveness ensures spatiotemporal control over drug release, significantly enhancing therapeutic efficacy while reducing systemic toxicity. Furthermore, the biodegradability of the nanogels allows for their breakdown into non-toxic byproducts, preventing long-term accumulation and reducing risks associated with persistent carriers like non-degradable micelles or inorganic nanoparticles. The system's ability to encapsulate both hydrophilic and hydrophobic drugs further broadens its applicability across various therapeutic agents and cancer types. By combining robust 5'-$VB_{12}$-mediated targeting, multistimuli responsiveness, and environmentally safe degradability, this technology represents a significant advancement in the field of nanomedicine, addressing critical limitations of existing drug delivery systems and offering enhanced specificity, efficacy, and safety for cancer therapy.

The present Inventors successfully developed and characterized a novel $VB_{12}$-modified monomer building block and its integration into functionalized nanogels, specifically designed for targeted and stimuli-responsive drug delivery. Using poly(N-isopropylacrylamide-co-acrylic acid) cross-linked with N,N'-bis(acryloyl)cystamine (BAC) and functionalized with 5'-$VB_{12}$ derivative, these nanogels demonstrated enhanced targeting capabilities due to the overexpression of CD320 receptors in some cancer cells. The $VB_{12}$-modified nanogels showed high encapsulation efficiency and controlled release of the anticancer drug doxorubicin (DOX), with degradation triggered by high concentration of glutathione (GSH) and pH 5, mimicking the tumor microenvironment.

[1] Sun, W., Qin, Y., & Zhang, C. (2024). Global cancer burden and challenges: Epidemiological insights. Journal of Hematology & Oncology, 17(1), Article 160. https://doi.org/10.1186/s13045-024-01640-8

[2] World Health Organization. (2020). Cancer. Retrieved from https://www.who.int/news-room/fact-sheets/detail/cancer

[3] Sung, H.; Ferlay, J.; Siegel, R. L.; Laversanne, M.; Soerjomataram, I.; Jemal, A.; Bray, F. Global Cancer Statistics 2020: GLOBOCAN Estimates of Incidence and Mortality Worldwide for 36 Cancers in 185 Countries. Ca-Cancer J. Clin. 2021, 71, 209- 249, DOI: 10.3322/caac.21660

[4] World Health Organization. Noncommunicable Diseases. Available at: https://www.who.int/news-room/fact-sheets/detail/noncommunicable-diseases (Accessed: 16.09.2023)

[5] Smith, B., & Jones, A. (2022). The socioeconomic impact of cancer: A comprehensive review of challenges and solutions. Healthcare, 10(12), Article 2370. https://doi.org/10.3390/healthcare10122370

[6] Siegel, R. L., Miller, K. D., & Jemal, A. (2020). Cancer statistics, 2020. CA: A Cancer Journal for Clinicians, 70(1), 7-30. https://doi.org/10.3322/caac.21590

[7] Baskar, R., Lee, K. A., Yeo, R., & Yeoh, K. W. (2012). Cancer and radiation therapy: Current advances and future directions. International Journal of Medical Sciences, 9(3), 193-199. https://doi.org/10.7150/ijms.3635

[8] National Cancer Institute. Types of Cancer Treatment. Available at: https://www.cancer.gov/about-cancer/treatment/types (Accessed: 23.08.2022).

[9] Al-Samkari, H., & Rizvi, M. A. (2019). The evolution of chemotherapy in the treatment of cancer. Oncology, 33(1), 1-12. https://doi.org/10.1200/JCO.2018.79.2964

[10] Shapiro, C. L., & Recht, A. (2001). Side effects of adjuvant treatment of breast cancer. The New England Journal of Medicine, 344(26), 1997-2008. https://doi.org/10.1056/NEJM200106283442607

[11] Shapiro, C. L., & Recht, A. (2001). Side effects of adjuvant treatment of breast cancer. The New England Journal of Medicine, 344(26), 1997-2008. https://doi.org/10.1056/NEJM200106283442607

[12] Zylberberg C, Matosevic S. Pharmaceutical liposomal drug delivery: a review of new delivery systems and a look at the regulatory landscape. Drug Deliv. 2016;23:3319-29.

[13] Cagel M, Tesan FC, Bernabeu E, Salgueiro MJ, Zubillaga MB, Moretton MA, et al. Polymeric mixed micelles as nanomedicines: achievements and perspectives. Eur J Pharm Biopharm. 2017;113:211-28. https://doi.org/10.1016/j.ejpb.2016.12.019.

[14] Patil Y, Shmeeda H, Amitay Y, Ohana P, Kumar S, Gabizon A. Targeting of folate-conjugated liposomes with co-entrapped drugs to prostate cancer cells via prostate-specific membrane antigen (PSMA). Nanomed Nanotechnol Biol Med. 2018; 14:1407-16. https://doi.org/10.1016/j.nano.2018.04.011.

[15] Zhang, T.; Yang, R.; Yang, S.; Guan, J.; Zhang, D.; Ma, Y.; Liu, H. Research Progress of Self-Assembled Nanogel and Hybrid Hydrogel Systems Based on Pullulan Derivatives. Drug Delivery 2018, 25, 278- 292, DOI: 10.1080/10717544.2018.1425776

[16] Langer, R., & Chasin, M. Biodegradable Polymers as Drug Delivery Systems. Marcel Dekker, Inc., 1990.

[17] Kaushik, N., Borkar, S.B., Nandanwar, S.K. et al. Nanocarrier cancer therapeutics with functional stimuli-responsive mechanisms. J Nanobiotechnol 20, 152 (2022). https://doi.org/10.1186/s12951-022-01364-2

[18] Akash Garg, Kamal Shah, Chetan singh Chauhan, Rutvi Agrawal, Ingenious nanoscale medication delivery system: Nanogel, Journal of Drug Delivery Science and Technology, Volume 92, 2024, 105289, ISSN 1773-2247, https://doi.org/10.1016/j.jddst.2023.105289.

[19] Mackiewicz, Marcin; Dagdelen, Serife; Waleka-Bargiel, Ewelina; Karbarz, Marcin, A polyampholyte core-shell microgel as an environmentally sensitive drug carrier, Arabian Journal of Chemistry, November 2023,17:105464, https://doi.org/10.1016/j.arabic.2023.105464

[20] De, I. R. R.; Aili, D.; Stevens, M. M. Enzyme-Responsive Nanoparticles for Drug Release and Diagnostics. Adv. Drug Delivery Rev. 2012, 64, 967- 978, DOI: 10.1016/j.addr.2012.01.002

[21] Huang, X. M.; Luo, Z. J.; Guo, J.; Ruan, Q. J.; Wang, J. M.; Yang, X. Q. Enzyme-Adsorbed Chitosan Nanogel Particles as Edible Pickering Interfacial Biocatalysts and Lipase-Responsive Phase Inversion of Emulsions. J. Agric. Food Chem. 2020, 68, 8890-8899, DOI: 10.1021/acs.jafc.0c00116

[22] Binauld, S.; Stenzel, M. H. Acid-Degradable Polymers for Drug Delivery: A Decade of Innovation. Chem. Commun. 2013, 49, 2082- 2102, DOI: 10.1039/c2cc36589h

[23] Ghassami, E.; Varshosaz, J.; Taymouri, S. Redox Sensitive Polysaccharide Based Nanoparticles for Improved Cancer Treatment: A Comprehensive Review. Curr. Pharm. Des. 2018, 24, 3303- 3319, DOI: 10.2174/1381612824666180813114841

[24] Sameiyan, E.; Bagheri, E.; Dehghani, S.; Ramezani, M.; Alibolandi, M.; Abnous, K.; Taghdisi, S. M. Aptamer-Based ATP-Responsive Delivery Systems for Cancer Diagnosis and Treatment. Acta Biomater. 2021, 123, 110-122, DOI: 10.1016/j.actbio.2020.12.057

[25] M. Mackiewicz, S. Dagdelen, M. Sagir Abubakar, J. Romanski, E. Waleka-Bargiel, M. Karbarz, Stimuli-sensitive and degradable capsules as drug carriers with decreased toxicity against healthy cells, Polymer Degradation and Stability, June 2023, 212:110349, https://doi.org/10.1016/j.polymdegradstab.2023.110349

[26] S. Dagdelen, M. Mackiewicz, M. Osial, E. Waleka-Bargiel, J. Romanski, P. Krysinski and M. Karbarz, Redox-responsive degradable microgel modified with superparamagnetic nanoparticles exhibiting controlled, hyperthermia-enhanced drug release, Journal of Materials Science, February 2023, 58:4094-4114, https://doi.org/10.1007/s10853-023-08168-1
[27] Jin, Z.; Wen, Y.; Hu, Y.; Chen, W.; Zheng, X.; Guo, W.; Wang, T.; Qian, Z.; Su, B. L.; He, Q. MRI-Guided and Ultrasound-Triggered Release of NO by Advanced Nanomedicine. Nanoscale 2017, 9, 3637- 3645, DOI: 10.1039/C7NR00231A
[28] Ai, X.; Mu, J.; Xing, B. Recent Advances of Light-Mediated Theranostics. Theranostics 2016, 6, 2439- 2457, DOI: 10.7150/thno.16088
[29] Huang, D.; Qian, H.; Qiao, H.; Chen, W.; Feijen, J.; Zhong, Z. Bioresponsive Functional Nanogels as an Emerging Platform for Cancer Therapy. Expert Opin. Drug Delivery 2018, 15, 703- 716, DOI: 10.1080/17425247.2018.1497607
[30] Li, F.; Liang, Z.; Ling, D. Smart Organic-Inorganic Nanogels for Activatable Theranostics. Curr. Med. Chem. 2019, 26, 1366-1376, DOI: 10.2174/0929867324666170920164614
[31] Hajebi, S.; Rabiee, N.; Bagherzadeh, M.; Ahmadi, S.; Rabiee, M.; Roghani-Mamaqani, H.; Tahriri, M.; Tayebi, L.; Hamblin, M. R. Stimulus-Responsive Polymeric Nanogels as Smart Drug Delivery Systems. Acta Biomater. 2019, 92, 1-18, DOI: 10.1016/j.actbio.2019.05.018
[32] Nichols, J. W., & Bae, Y. H. (2014). EPR: Evidence and fallacy. Journal of Controlled Release, 190, 451-464. https://doi.org/10.1016/j.jconrel.2014.03.057
[33] Danhier, F. (2016). To exploit the tumor microenvironment: Since the EPR effect fails in the clinic, what is the future of nanomedicine? Journal of Controlled Release, 244, 108-121. https://doi.org/10.1016/j.jconrel.2016.11.015
[34] Wilhelm, S., Tavares, A. J., Dai, Q., Ohta, S., Audet, J., Dvorak, H. F., & Chan, W. C. W. (2016). Analysis of nanoparticle delivery to tumours. Nature Reviews Materials, 1(5), 16014. https://doi.org/10.1038/natrevmats.2016.14
[31] Cheng, C.J. et al. A holistic approach to targeting disease with polymeric nanoparticles. Nat. Rev. Drug Discov. 14, 239-247 (2015).
[36] Natalia V. Nukolova, Hardeep S. Oberoi, Samuel M. Cohen, Alexander V. Kabanov, Tatiana K. Bronich, Folate-decorated nanogels for targeted therapy of ovarian cancer, Biomaterials, 2011, DOI: 10.1016/j.biomaterials.2011.04.006
[37] Narwade, M., Shaikh, A., Gajbhiye, K.R. et al. Advanced cancer targeting using aptamer functionalized nanocarriers for site-specific cargo delivery. Biomater Res 27, 42 (2023). https://doi.org/10.1186/s40824-023-00365-y
[38] Rodriguez-Nava C, Ortuño-Pineda C, Illades-Aguiar B, Flores-Alfaro E, Leyva-Vázquez MA, Parra-Rojas I, Del Moral-Hernández O, Vences-Velázquez A, Cortés-Sarabia K, Alarcón-Romero LDC. Mechanisms of Action and Limitations of Monoclonal Antibodies and Single Chain Fragment Variable (scFv) in the Treatment of Cancer. Biomedicines. 2023 Jun 1;11(6):1610. doi: 10.3390/biomedicines11061610. PMID: 37371712; PMCID: PMC10295864.
[39] Patel, K., Sobczyńska-Malefora, A. The adverse effects of an excessive folic acid intake. Eur J Clin Nutr 71, 159-163 (2017). https://doi.org/10.1038/ejcn.2016.194
[40] Ishiwata K, Takahashi T, Iwata R et al. Tumor diagnosis by PET: potential of seven tracers examined in five experimental tumors including an artificial metastasis model. Int. J. Rad. Appl. Instrum. B 19(6), 611-618 (1992).
[41] Clardy SM, Allis DG, Fairchild TJ, Doyle RP. Vitamin B12 in drug delivery: breaking through the barriers to a B12 bioconjugate pharmaceutical. Expert Opin. Drug Deliv. 8(1), 127-140 (2011).
[42] Arora K, Sequeira JM, Quadros EV. Maternofetal transport of vitamin B12: role of TCbIR/CD320 and megalin. FASEB J. 31(7), 3098-3106 (2017).
[43] Seetharam B. Receptor-mediated endocytosis of cobalamin (vitamin B12). Annu. Rev. Nutr. 19, 173-195 (1999).
[44] Quadros, E. V., Nakayama, Y. & Sequeira, J. M. The protein and the gene encoding the receptor for the cellular uptake of transcobalamin-bound cobalamin. Blood 113, 186-192 (2009). [45] Quadros, E. V. Advances in the understanding of cobalamin assimilation and metabolism. Br. J. Haematol. 148, 195-204 (2010).
[46] Jiang, W., Nakayama, Y., Sequeira, J. M. & Quadros, E. V. Mapping the functional domains of TCblR/CD320, the receptor for cellular uptake of transcobalamin-bound cobalamin. FASEB J. 27, 2988-2994 (2013).
[47] Willnow, T. E. et al. RAP, a specialized chaperone, prevents ligand-induced ER retention and degradation of LDL receptor-related endocytic receptors. EMBO J. 15, 2632-2639 (1996).
[48] F. Zelder "Recent trends in the development of vitamin B12 derivatives for medicinal applications" Chem. Commun. 2015, 51, 14004-14017.
[49] Clardy SM, Allis DG, Fairchild TJ, Doyle RP. Vitamin B12 in drug delivery: breaking through the barriers to a B12 bioconjugate pharmaceutical. Expert Opinion on Drug Delivery. 2011 Jan;8(1):127-140. DOI: 10.1517/17425247.2011.539200. PMID: 21128823.
[50] Gherasim, C., Lofgren, M., & Banerjee, R. (2013). Navigating the B12 road: Assimilation, delivery, and disorders of cobalamin. Journal of Biological Chemistry, 288(19), 13186-13193. https://doi.org/10.1074/jbc.R113.458810
[51] Thepphankulngarm, N., Wonganan, P., Sapcharoenkun, C., Tuntulani, T., & Leeladee, P. (2017). Combining vitamin B12 and cisplatin-loaded porous silica nanoparticles via coordination: A facile approach to prepare a targeted drug delivery system. New Journal Chemistry, 41(22), 13823-13829. https://doi.org/10.1039/C7NJ02754K
[52] Smith, J. A., & Doe, L. M. (2024). A review of coordination compounds: Structure, stability, and applications. Reviews in Inorganic Chemistry, 35(2), 123-145. https://doi.org/10.1515/revic-2024-0035

[53] Guo, W., Deng, L., Chen, Z., Chen, Z., Yu, J., Liu, H., ... Hu, Y. (2018). Vitamin-B12-conjugated sericin micelles for targeting CD320-overexpressed gastric cancer and reversing drug resistance. Nanomedicine. doi:10.2217/nnm-2018-0321

[54] Drozdov, A. S., Nikitin, P. I., & Rozenberg, J. M. (2021). Systematic Review of Cancer Targeting by Nanoparticles Revealed a Global Association between Accumulation in Tumors and Spleen. International Journal of Molecular Sciences, 22(23), 13011. https://doi.org/10.3390/ijms222313011

[55] Gamcsik MP, Kasibhatla MS, Teeter SD, Colvin OM. Glutathione levels in human tumors. Biomarkers. 2012 Dec;17(8):671-91. doi: 10.3109/1354750X.2012.715672. Epub 2012 Aug 20. PMID: 22900535; PMCID: PMC3608468.

[56] Patra, Jayanta Kumar; Das, Gitishree; Fraceto, Leonardo Fernandes; Campos, Estefania Vangelie Ramos; Rodriguez-Torres, Maria del Pilar; Acosta-Torres, Laura Susana; Diaz-Torres, Luis Armando; Grillo, Renato; Swamy, Mallappa Kumara; Sharma, Shivesh; Habtemariam, Solomon (December 2018). "Nano based drug delivery systems: recent developments and future prospects". Journal of Nanobiotechnology. 16 (1): 71. doi:10.1186/s12951-018-0392-8. ISSN 1477-3155. PMC 6145203. PMID 30231877

[57] Li, Yulin; Maciel, Dina; Rodrigues, João; Shi, Xiangyang; Tomás, Helena (2015-08-26). "Biodegradable Polymer Nanogels for Drug/Nucleic Acid Delivery". Chemical Reviews. 115 (16): 8564-8608. doi:10.1021/cr500131f. ISSN 0009-2665. PMID 26259712. S2CID 1651110.

[58] Glutathione-Sensitive Nanogels for Drug Release, Giulio Ghersi and Clelia Dispenza and Marianna Sabatino and Natascia Grimaldi and Giorgia Adamo and Simona Campora, Chemical engineering transactions, 2014, 38

[59] Jackowska, A. and Chromiński, M. and Giedyk, M. and Gryko, D., 5'-Vitamin B12 derivatives suitable for bioconjugation via the amide bond, Org. Biomol. Chem., 2018,16, 936-943 [60] Navigating the B12 Road: Assimilation, Delivery, and Disorders of Cobalamin * Gherasim, Carmen et al. Journal of Biological Chemistry, Volume 288, Issue 19, 13186 - 13193

[61] Wuerges, Jochen & Geremia, Silvano & Fedosov, Sergey & Randaccio, Lucio. (2007). Vitamin B12 Transport Proteins: Crystallographic Analysis of β-axial Ligand Substitutions in Cobalamin Bound to Transcobalamin. IUBMB life. 59. 722-9. 10.1080/15216540701613413.

[62] Horton, R. A.; Bagnato, J. D.; Grissom, C. B. Structural Determination of 5'-OH α-Ribofuranoside Modified Cobalamins via 13C and DEPT NMR. J. Org. Chem. 2003, 68 (18), 7108-7111. https://doi.org/10.1021/jo0340399

[63] Bellamy, L. J. (1980). The Infrared Spectra of Complex Molecules. Springer.

[64] Smith, B. (1998). Infrared Spectral Interpretation: A Systematic Approach. CRC Press.

[65] Nakamoto, K. (2008). Infrared and Raman Spectra of Inorganic and Coordination Compounds: Part A: Theory and Applications in Inorganic Chemistry. Wiley.

[66] Socrates, G. (2001). Infrared and Raman characteristic group frequencies: Tables and charts (3rd ed.). Wiley.

[67] Colthup, N. B., Daly, L. H., & Wiberley, S. E. (1990). Introduction to infrared and Raman spectroscopy (3rd ed.). Academic Press.

[68] Saunders, B. R., & Vincent, B. (1999). Advances in Colloid and Interface Science, 80(1), 1-25.

[69] Pelton, R. (2000). Colloids and Surfaces A: Physicochemical and Engineering Aspects, 142(2-3), 171-181

[70] Schild, H. G., & Tirrell, D. A. (1990). Langmuir, 6(9), 2010-2017.

[71] Robert J. Hunter, Zeta Potential in Colloid Science: Principles and Applications, Academic Press, 1981

[72] Berne, B. J., & Pecora, R. (2000). Dynamic Light Scattering: With Applications to Chemistry, Biology, and Physics. Dover Publications.

[73] Rosenholm, J. M., Meinander, A., Peussa, M., & Lindén, M. (2011). Dynamic light scattering (DLS) and small-angle X-ray scattering (SAXS) studies of the size distribution of monodisperse silica nanoparticles. Journal of Colloid and Interface Science, 362(1), 16-23.

[74] Sussana A. Elkassih, Petra Kos, Hu Xionga and Daniel J. Siegwart, Degradable redox-responsive disulfide-based nanogel drug carriers via dithiol oxidation polymerization, Biomater. Sci., 2019,7, 607-617.

[75] Giustarini D, Milzani A, Dalle-Donne I, Rossi R. How to Increase Cellular Glutathione. Antioxidants (Basel). 2023 May 13;12(5):1094. doi: 10.3390/antiox12051094. PMID: 37237960; PMCID: PMC10215789

[76] Ru Cheng, Fang Feng, Fenghua Meng, Chao Deng, Jan Feijen, Zhiyuan Zhong, Glutathione-responsive nano-vehicles as a promising platform for targeted intracellular drug and gene delivery, Journal of Controlled Release, Volume 152, Issue 1, 2011, Pages 2-12, ISSN 0168-3659, https://doi.org/10.1016/j.jconrel.2011.01.030

[77] West K.R., Otto S., 2005, Reversible covalent chemistry in drug delivery, Curr. Drug Discov. Technol., 2:123-160..

[78] Gamcsik MP, Kasibhatla MS, Teeter SD, Colvin OM. Glutathione levels in human tumors. Biomarkers. 2012 Dec;17(8):671-91. doi: 10.3109/1354750X.2012.715672. Epub 2012 Aug 20. PMID: 22900535; PMCID: PMC3608468.

[79] American Association for Cancer Research. (2021). Simultaneous knockdown of CD320 and LRP2 receptors significantly inhibits cell proliferation in multiple cancer cell lines, including breast cancer. Cancer Research, 81(13_Supplement), Abstract 1223. https://doi.org/10.1158/1538-7445.AM2021-1223

## Claims

1. Vitamin B12 monomer having the structure represented by the formula 4

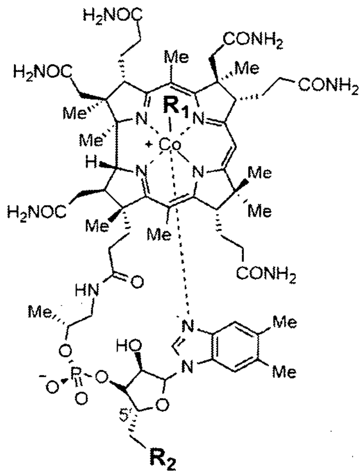

4

wherein:

$R_1$ is selected from the group $C_{15}H_{22}N_6O_5S$, $CH_3$, OH, CN, and
$R_2$ is selected from the group comprising carbamate, alkyl, aryl, amide, ester, thiol, or polymeric chains.

2. Vitamin B12 monomer according to claim 1 having the structure represented by the formula 3

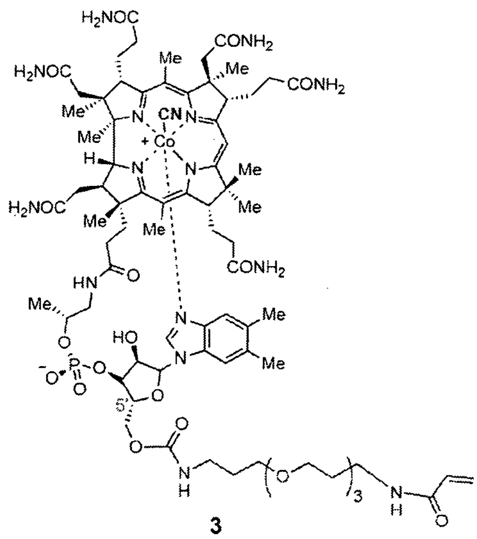

3

3. Method of synthesis of Vitamin B12 monomer having the structure represented by the formula 3 or 4 comprising

in step one

- activation of the Cyanocobalamin by activation of the 5'-hydroxyl group of the ribose moiety, wherein the activation is achieved through esterification, tosylation, halogenation, or similar reactions, and

in step two

- conjugation using versatile reactions such as carbamate formation, nucleophilic substitution, click chemistry, or amide bond formation.

**4.** Method of synthesis of Vitamin B12 monomer having the structure represented by the formula 3 according to claim 3 comprising:

in step one

- activation of the Cyanocobalamin at the 5' hydroxide (-OH) ribose position using CDT;
- carbamate coupling reaction of the previously 5'-activated Cyanocobalamin with 4,7,10-trioxa-1,13-tridecadiamine and DIPEA with stirring and heating;
- precipitation of the reaction product and purification; and

in step two

- dissolution of the reaction product obtained in step one in DMF;
- reaction of the dissolved product with N,N-Diisopropylethylamine and acryloyl chloride at 0°C;
- precipitation of the reaction product and purification.

**5.** Method of claim 4, wherein the reaction is carried out in DMSO under gentle heating.

**6.** Method of claim 4 or 5, wherein acryloyl chloride is added 30 minutes after N,N-Diisopropylethylamine.

**7.** Method of synthesis of nanogels modified with Vitamin B12 using precipitation polymerization, comprising:

- dissolution of a main monomer NIPA, a comonomer AA and a linker BAC in deionized water,
- heating and deoxygenating of the reaction mixture;
- addition of APS and TEMED to initiate the reaction;
- addition of the Vitamin B12 monomer and stirring the reaction mixture;
- cooling the reaction mixture to the room temperature;
- precipitation of the reaction product and purification.

**8.** Method of claim 7, wherein the pH is adjusted to 5.0, and/or the reaction mixture is heated to 55°C, and/or the reaction mixture is deoxygenated for 0.5 h using argon.

**9.** Method of any of claims 7-8, wherein the Vitamin B12 monomer is selected form the monomers defined in claim 1 or 2, or obtained by the method defined in claims 3-6.

**10.** Use of the nanogel modified with Vitamin B12 in drug delivery systems.

**11.** Nanogel modified with Vitamin B12 for use in the treatment.

**12.** Nanogel modified with Vitamin B12 for use in the treatment of cancer.

**13.** Pharmaceutical composition comprising nanogel modified with Vitamin B12 and active ingredient.

**14.** Pharmaceutical composition according to claim 13, wherein the active ingredient is anticancer drug, such as doxorubicine.

**15.** Pharmaceutical composition comprising nanogel modified with Vitamin B12 and active ingredient for use in the treatment of cancer.

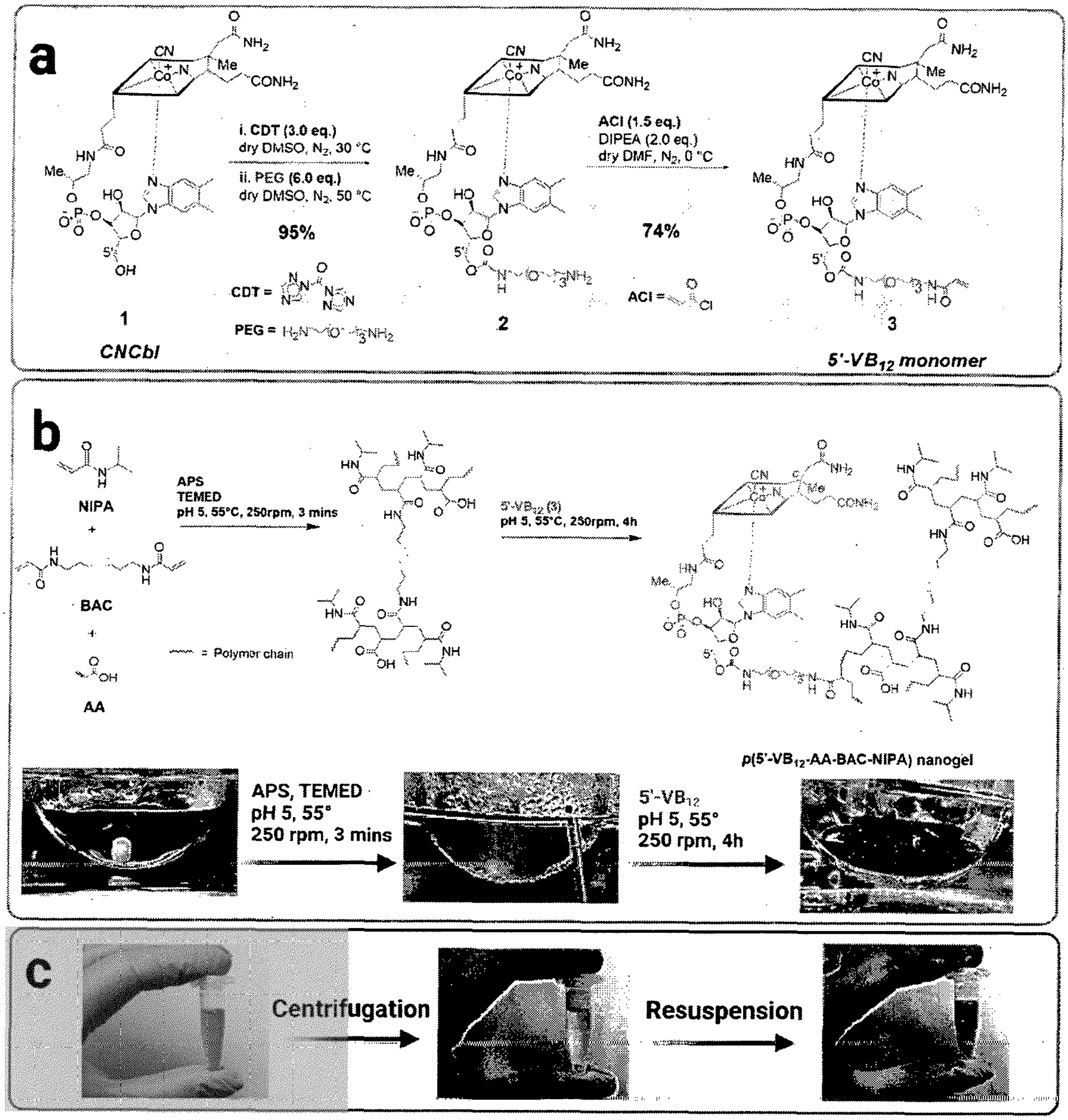
a
i. CDT (3.0 eq.)
dry DMSO, $N_2$, 30 °C
ii. PEG (6.0 eq.)
dry DMSO, $N_2$, 50 °C
95%
ACl (1.5 eq.)
DIPEA (2.0 eq.)
dry DMF, $N_2$, 0 °C
74%
CDT =
PEG =
ACl =
1
CNCbl
2
3
5'-$VB_{12}$ monomer
b
NIPA
BAC
AA
APS
TEMED
pH 5, 55°C, 250rpm, 3 mins
= Polymer chain
5'-$VB_{12}$ (3)
pH 5, 55°C, 250rpm, 4h
p(5'-$VB_{12}$-AA-BAC-NIPA) nanogel
APS, TEMED
pH 5, 55°
250 rpm, 3 mins
5'-$VB_{12}$
pH 5, 55°
250 rpm, 4h
c
Centrifugation
Resuspension

**FIG. 1.**

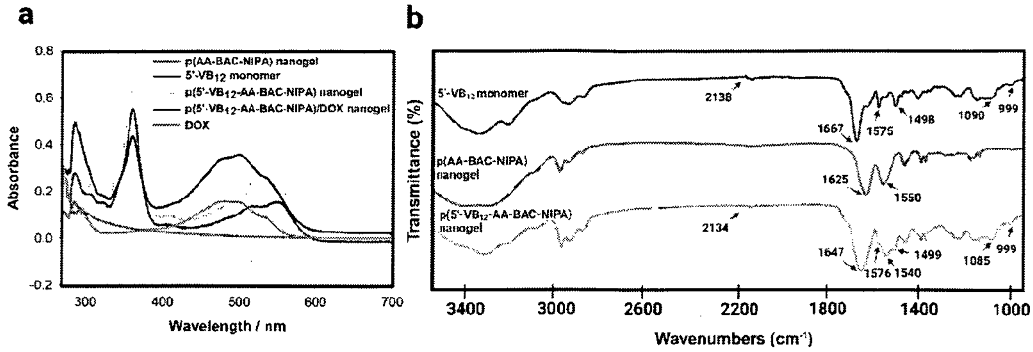


FIG. 2.

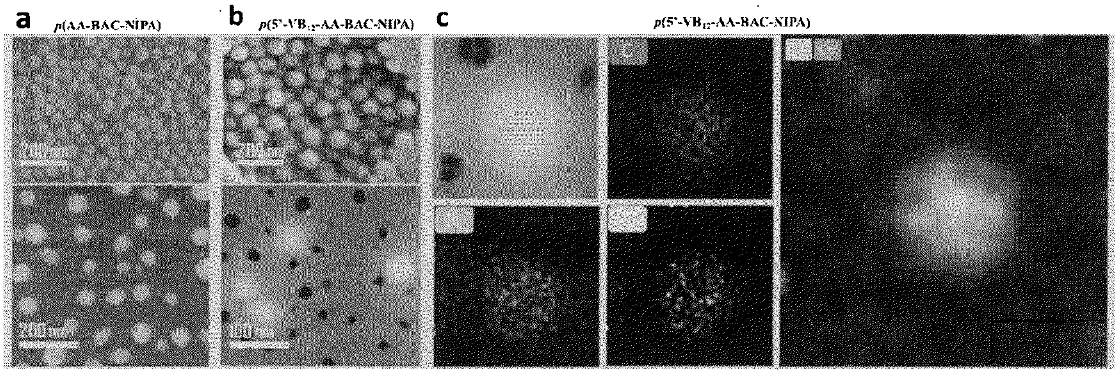


FIG. 3.

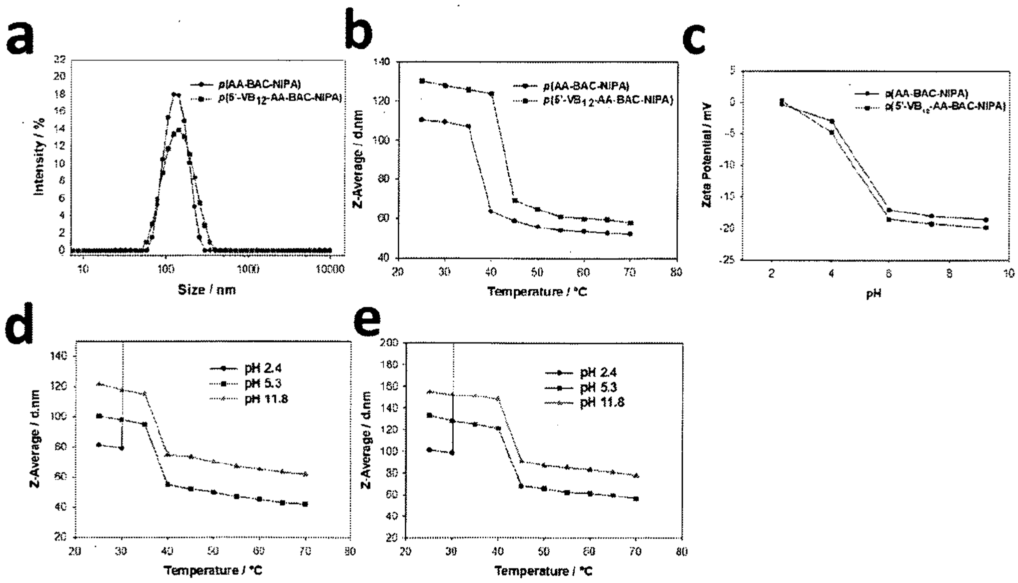


FIG. 4.

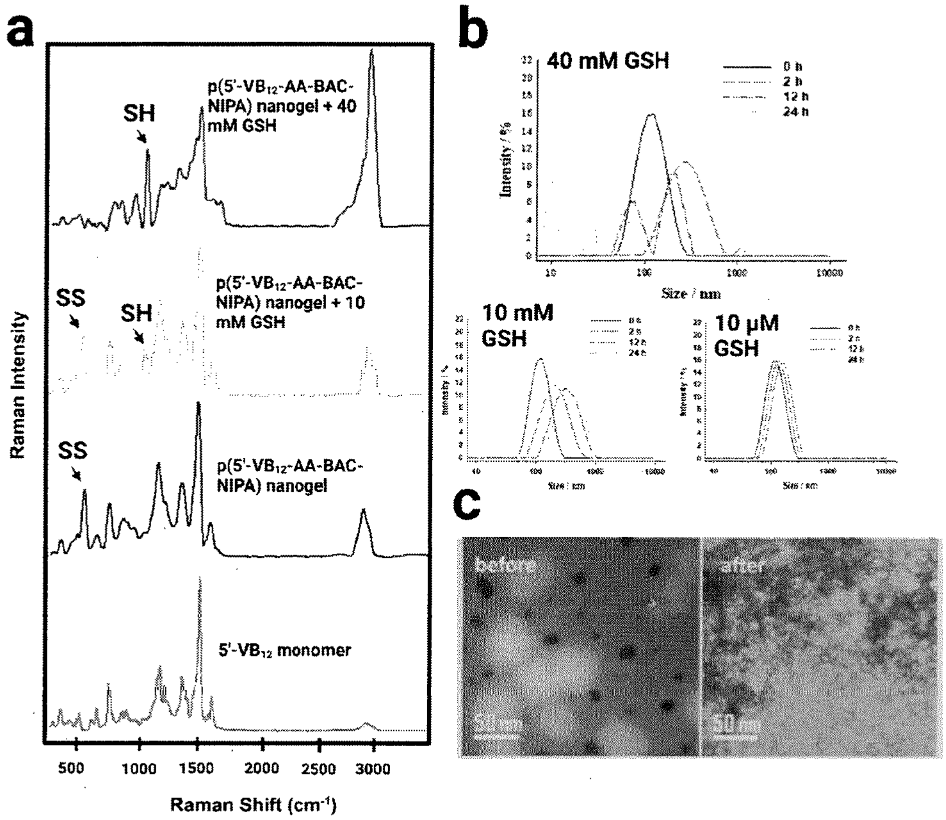
a
b
c
Raman Intensity
Raman Shift (cm⁻¹)
SH
SS
p(5'-VB12-AA-BAC-NIPA) nanogel + 40 mM GSH
p(5'-VB12-AA-BAC-NIPA) nanogel + 10 mM GSH
p(5'-VB12-AA-BAC-NIPA) nanogel
5'-VB12 monomer
40 mM GSH
10 mM GSH
10 μM GSH
0 h
2 h
12 h
24 h
Intensity / %
Size / nm
before
after
50 nm

FIG. 5.

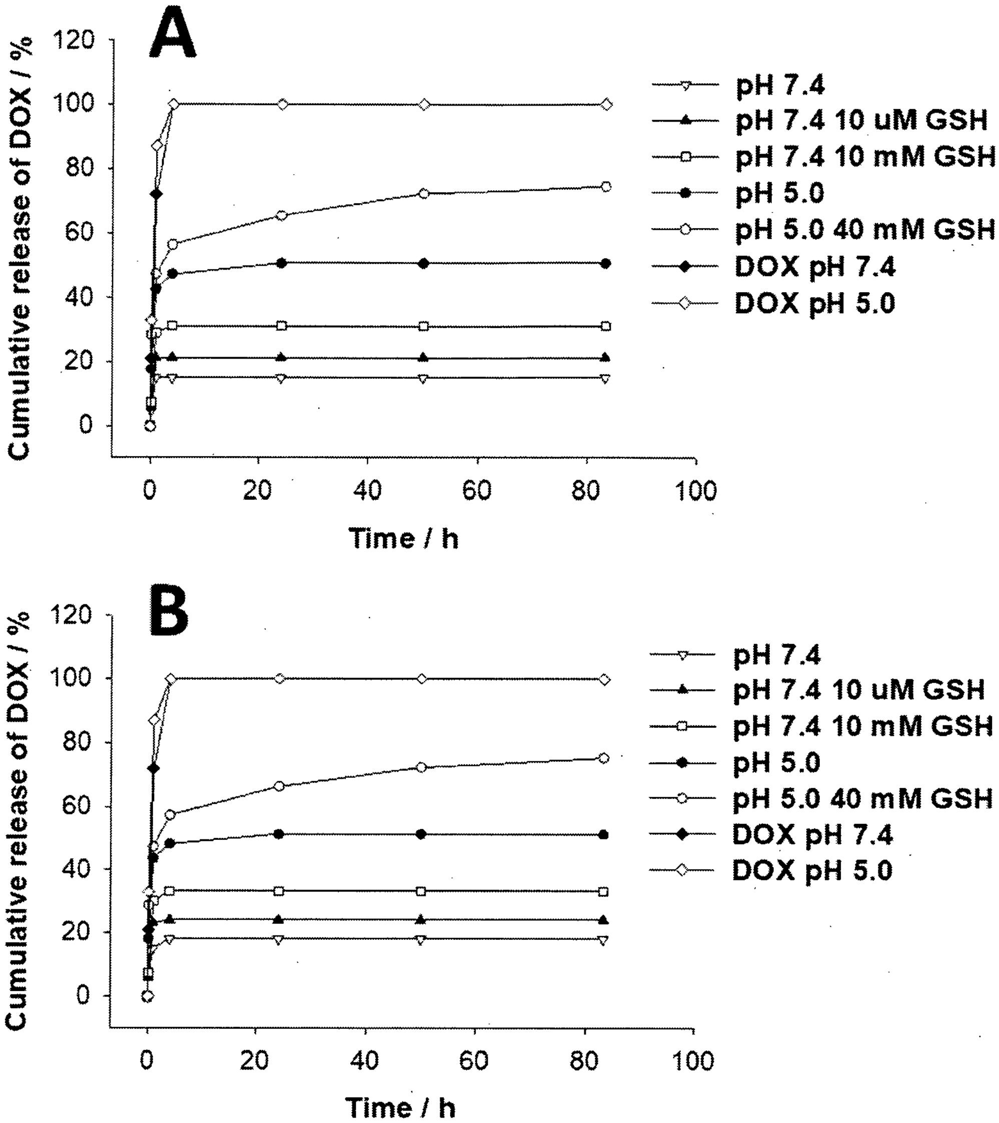
A
Cumulative release of DOX / %
Time / h
pH 7.4
pH 7.4 10 uM GSH
pH 7.4 10 mM GSH
pH 5.0
pH 5.0 40 mM GSH
DOX pH 7.4
DOX pH 5.0
B
Cumulative release of DOX / %
Time / h
pH 7.4
pH 7.4 10 uM GSH
pH 7.4 10 mM GSH
pH 5.0
pH 5.0 40 mM GSH
DOX pH 7.4
DOX pH 5.0

FIG. 6.

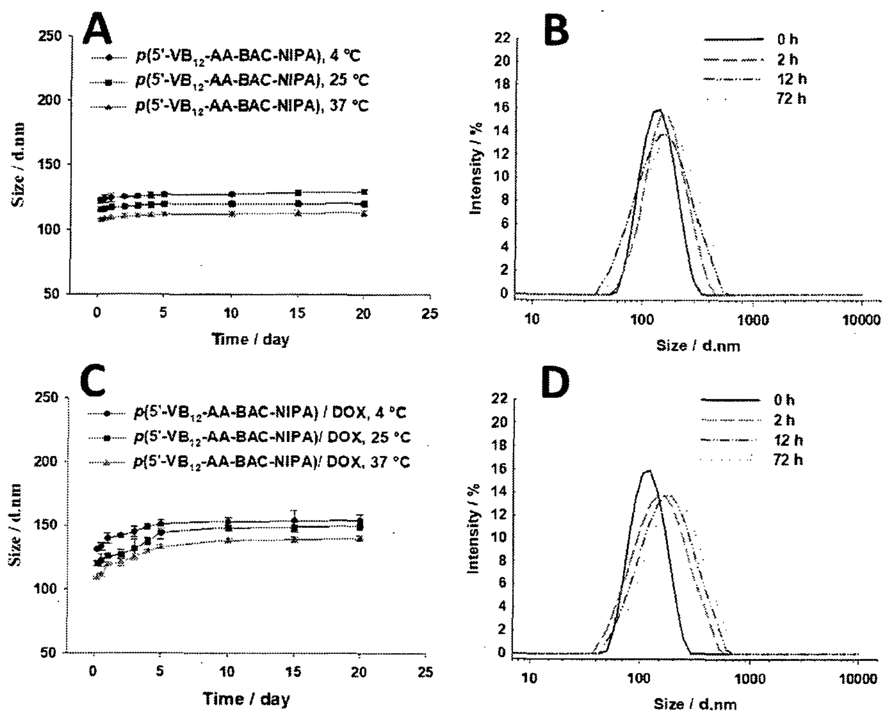
A
p(5'-VB12-AA-BAC-NIPA), 4 °C
p(5'-VB12-AA-BAC-NIPA), 25 °C
p(5'-VB12-AA-BAC-NIPA), 37 °C
Size / d.nm
Time / day
B
0 h
2 h
12 h
72 h
Intensity / %
Size / d.nm
C
p(5'-VB12-AA-BAC-NIPA) / DOX, 4 °C
p(5'-VB12-AA-BAC-NIPA)/ DOX, 25 °C
p(5'-VB12-AA-BAC-NIPA)/ DOX, 37 °C
Size / d.nm
Time / day
D
0 h
2 h
12 h
72 h
Intensity / %
Size / d.nm

**FIG. 7.**

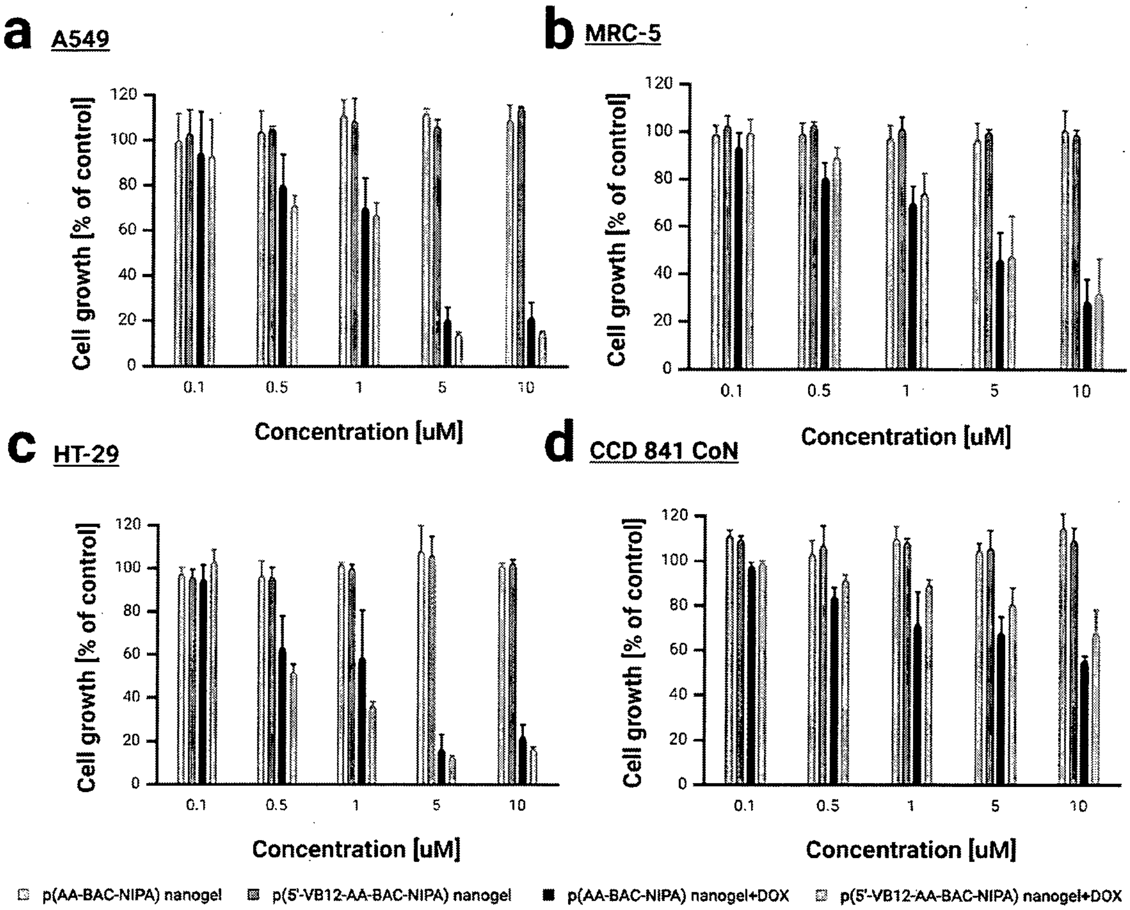
a A549
b MRC-5
c HT-29
d CCD 841 CoN
Cell growth [% of control]
Concentration [uM]
0
20
40
60
80
100
120
0.1
0.5
1
5
10
p(AA-BAC-NIPA) nanogel
p(5'-VB12-AA-BAC-NIPA) nanogel
p(AA-BAC-NIPA) nanogel+DOX
p(5'-VB12-AA-BAC-NIPA) nanogel+DOX

FIG. 8.

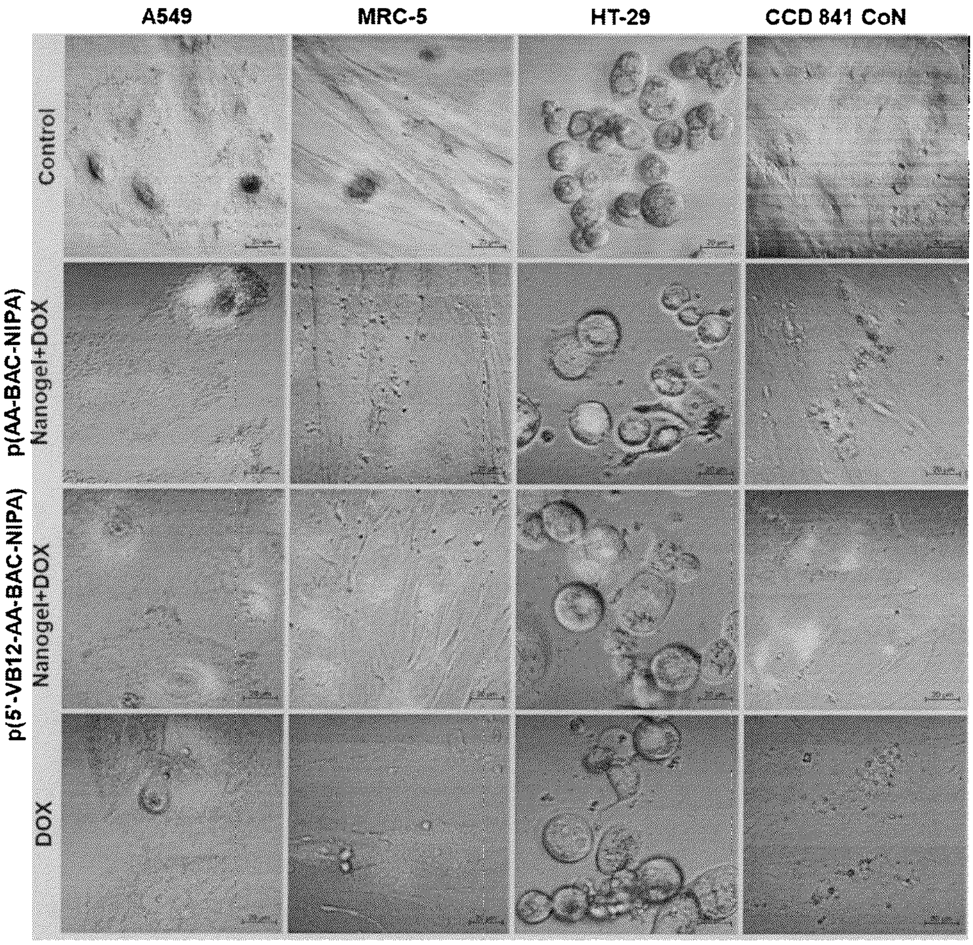
A549
MRC-5
HT-29
CCD 841 CoN
Control
p(AA-BAC-NIPA) Nanogel+DOX
p(5'-VB12-AA-BAC-NIPA) Nanogel+DOX
DOX

FIG. 9.

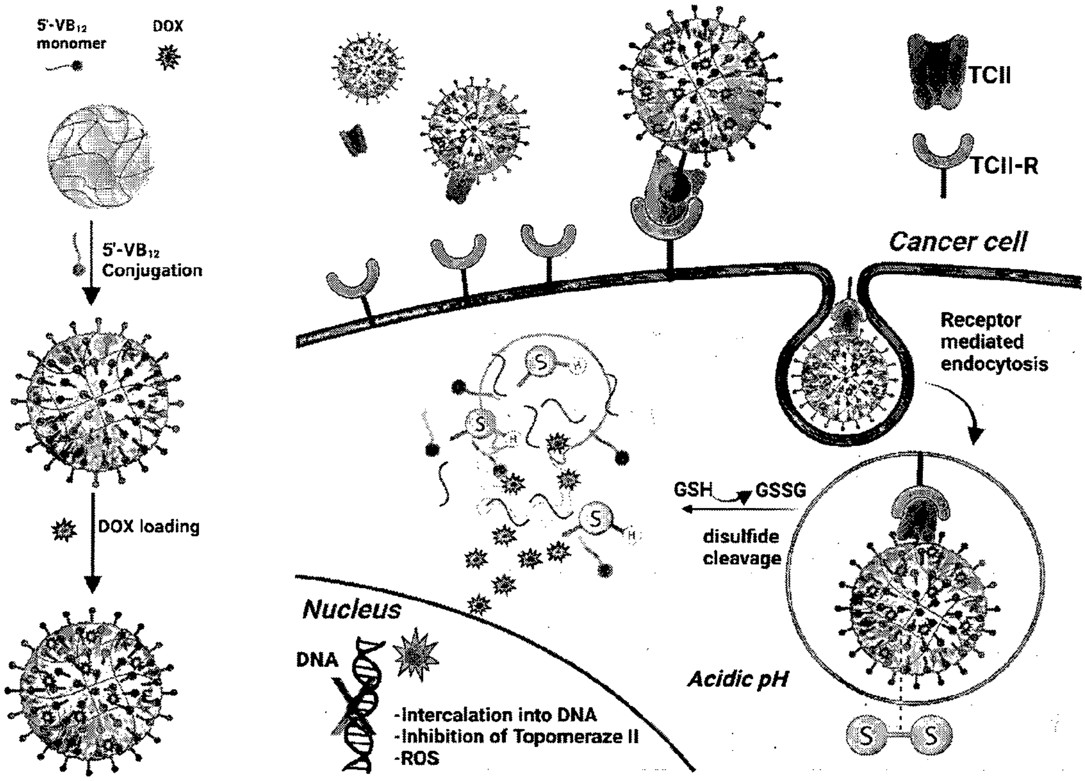
5'-VB12 monomer
DOX
5'-VB12 Conjugation
DOX loading
TCII
TCII-R
Cancer cell
Receptor mediated endocytosis
GSH
GSSG
disulfide cleavage
Acidic pH
Nucleus
DNA
-Intercalation into DNA
-Inhibition of Topomeraze II
-ROS

FIG. 10.

Europäisches Patentamt
European Patent Office
Office européen des brevets

# EUROPEAN SEARCH REPORT

Application Number
EP 25 46 0005

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GUPTA Y ET AL: "Vitamin B12-mediated transport: A potential tool for tumor targeting of antineoplastic drugs and imaging agents", CRITICAL REVIEWS IN THERAPEUTIC DRUG CARRIER SYSTEMS, BEGELL HOUSE PUBLISHING INC, US, vol. 25, no. 4, 1 January 2008 (2008-01-01), pages 347-379, XP008121962, ISSN: 0743-4863 | 1,3 | INV. C07H23/00 C07H1/00 A61P35/00 B82Y5/00 B82Y40/00 C08F2/10 A61K47/32 |
| Y | * page 359 - page 362 * | 10-15 | |
| A | | 2,7-9 | |
| | ----- | | |
| X | WO 2012/122313 A2 (ACCESS PHARMA INC [US]; NOWOTNIK DAVID P [US] ET AL.) 13 September 2012 (2012-09-13) | 1,3 | |
| Y | * page 53 - page 65; figure 2 * | 10-15 | |
| A | | 2,7-9 | |
| | ----- | | TECHNICAL FIELDS SEARCHED (IPC) |
| X | WO 2011/130716 A2 (ACCESS PHARMA INC [US]; ZARZYCKI RYSZARD [US] ET AL.) 20 October 2011 (2011-10-20) | 1,3 | C07H C08F B82Y A61P |
| Y | * page 54 - page 61; figure 2 * | 10-15 | |
| A | | 2,7-9 | |
| | ----- | | |
| X | CHALASANI ET AL: "Effective oral delivery of insulin in animal models using vitamin B12-coated dextran nanoparticles", JOURNAL OF CONTROLLED RELEASE, ELSEVIER, NL, vol. 122, no. 2, 5 September 2007 (2007-09-05), pages 141-150, XP022230608, ISSN: 0168-3659, DOI: 10.1016/J.JCONREL.2007.05.019 | 1,3 | |
| Y | * page 143; figure 1; table 1 * | 10-15 | |
| A | | 2,7-9 | |
| | ----- | | |
| | -/-- | | |

The present search report has been drawn up for all claims

2

| Place of search | Date of completion of the search | Examiner |
|---|---|---|
| Munich | 1 July 2025 | Zamarija, Ivica |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 5

Europäisches Patentamt
European Patent Office
Office européen des brevets

# EUROPEAN SEARCH REPORT

Application Number
EP 25 46 0005

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Ruiz-sánchez Pilar: "Vitamin B 12 : A Potential Targeting Molecule for Therapeutic Drug Delivery : Where Chemistry Meets Life" In: "Ideas in Chemistry and Molecular Sciences : Where Chemistry Meets Life", 24 March 2010 (2010-03-24), Wiley, XP093289386, ISBN: 978-3-527-63051-6 pages 93-115, DOI: 10.1002/9783527630516.ch4, Retrieved from the Internet: URL:https://onlinelibrary.wiley.com/doi/pdf/10.1002/9783527630516.ch4> | 1,3 | |
| Y | * page 104; figure 4.6 * | 10-15 | |
| A | | 2,7-9 | |
| | ----- | | |
| X | BLOCH JOËL S. ET AL: "Generation of nanobodies targeting the human, transcobalamin-mediated vitamin B 12 uptake route", THE FASEB JOURNAL, vol. 36, no. 4, 26 February 2022 (2022-02-26), XP093289117, US ISSN: 0892-6638, DOI: 10.1096/fj.202101376RR Retrieved from the Internet: URL:https://onlinelibrary.wiley.com/doi/full-xml/10.1096/fj.202101376RR> | 1,3 | TECHNICAL FIELDS SEARCHED (IPC) |
| Y | * page 2 - page 4 * | 10-15 | |
| A | | 2,7-9 | |
| | ----- | | |
| | -/-- | | |

The present search report has been drawn up for all claims

2

| Place of search | Date of completion of the search | Examiner |
|---|---|---|
| Munich | 1 July 2025 | Zamarija, Ivica |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 5

Europäisches Patentamt
European Patent Office
Office européen des brevets

# EUROPEAN SEARCH REPORT

Application Number
EP 25 46 0005

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GUO WEIHONG ET AL: "Vitamin B12-conjugated Sericin Micelles for Targeting CD320-overexpressed Gastric Cancer and Reversing Drug Resistance", NANOMEDICINE, vol. 14, no. 3, 17 October 2018 (2018-10-17), pages 353-370, XP093289116, ISSN: 1743-5889, DOI: 10.2217/nnm-2018-0321 | 1,3 | |
| Y | * figure 7 * | 10-15 | |
| A | | 2,7-9 | |
| | ----- | | |
| X | DELASOIE JOACHIM ET AL: "Slow-targeted release of a ruthenium anticancer agent from vitamin B 12 functionalized marine diatom microalgae", DALTON TRANSACTION, vol. 47, no. 48, 1 January 2018 (2018-01-01), pages 17221-17232, XP093289486, UK ISSN: 1477-9226, DOI: 10.1039/C8DT02914H | 1,3-6 | |
| Y | * pages 17226-17227 (Figures 3-4) * | 10-15 | |
| A | | 2,7-9 | |
| | ----- | | |
| X | HORTON ROBERT A. ET AL: "Structural Determination of 5'-OH [alpha]-Ribofuranoside Modified Cobalamins via 13 C and DEPT NMR", THE JOURNAL OF ORGANIC CHEMISTRY, vol. 68, no. 18, 9 August 2003 (2003-08-09), pages 7108-7111, XP093289485, United States ISSN: 0022-3263, DOI: 10.1021/jo0340399 * page 7110 (Scheme 1) * | 1,3-6 | |
| | ----- | | |
| | -/-- | | |

TECHNICAL FIELDS SEARCHED (IPC)

The present search report has been drawn up for all claims

2

| Place of search | Date of completion of the search | Examiner |
|---|---|---|
| Munich | 1 July 2025 | Zamarija, Ivica |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 25 46 0005

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | RODGERS ZACHARY L. ET AL: "B 12 -Mediated, Long Wavelength Photopolymerization of Hydrogels", LANGMUIR, vol. 137, no. 9, 11 March 2015 (2015-03-11), pages 3372-3378, XP055798407, DOI: 10.1021/jacs.5b00182 Retrieved from the Internet: URL:https://www.ncbi.nlm.nih.gov/pmc/articles/PMC4610811/pdf/nihms729520.pdf> * pages 5 and 17 (Scheme 1) * | 1,3-6 | |
| Y | SABIT HUSSEIN ET AL: "Nanocarriers: A Reliable Tool for the Delivery of Anticancer Drugs", PHARMACEUTICS, vol. 14, no. 8, 28 July 2022 (2022-07-28), page 1566, XP093289565, Switzerland ISSN: 1999-4923, DOI: 10.3390/pharmaceutics14081566 | 10-15 | TECHNICAL FIELDS SEARCHED (IPC) |
| A | * the whole document * | 2,7-9 | |
| Y | VARGASON AVA M ET AL: "The evolution of commercial drug delivery technologies", NATURE BIOMEDICAL ENGINEERING, NATURE PUBLISHING GROUP UK, LONDON, vol. 5, no. 9, 1 April 2021 (2021-04-01), pages 951-967, XP037581374, DOI: 10.1038/S41551-021-00698-W [retrieved on 2021-04-01] | 10-15 | |
| A | * the whole document * | 2,7-9 | |

-/--

The present search report has been drawn up for all claims

| Place of search | Date of completion of the search | Examiner |
|---|---|---|
| Munich | 1 July 2025 | Zamarija, Ivica |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 4 of 5

Europäisches Patentamt
European Patent Office
Office européen des brevets

# EUROPEAN SEARCH REPORT

Application Number
EP 25 46 0005

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | FARRAN BATOUL ET AL: "Folate-conjugated nanovehicles: Strategies for cancer therapy", MATERIALS SCIENCE AND ENGINEERING C, ELSEVIER SCIENCE S.A, CH, vol. 107, 23 October 2019 (2019-10-23), XP085917442, ISSN: 0928-4931, DOI: 10.1016/J.MSEC.2019.110341 [retrieved on 2019-10-23] | 10-15 | |
| A | * page 1, last line ff.; page 5, line 9 of column "5." * | 2,7-9 | |
| | ----- | | |

TECHNICAL FIELDS SEARCHED (IPC)

The present search report has been drawn up for all claims

| Place of search | Date of completion of the search | Examiner |
|---|---|---|
| Munich | 1 July 2025 | Zamarija, Ivica |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO.

EP 25 46 0005

This annex lists the patent family members relating to the patent documents cited in the above-mentioned European search report. The members are as contained in the European Patent Office EDP file on
The European Patent Office is in no way liable for these particulars which are merely given for the purpose of information.

01-07-2025

| Patent document cited in search report | Publication date | Patent family member(s) | Publication date |
|---|---|---|---|
| WO 2012122313 A2 | 13-09-2012 | CN 103501821 A | 08-01-2014 |
| | | EP 2683410 A2 | 15-01-2014 |
| | | KR 20140026396 A | 05-03-2014 |
| | | US 2012231069 A1 | 13-09-2012 |
| | | US 2016175259 A1 | 23-06-2016 |
| | | WO 2012122313 A2 | 13-09-2012 |
| WO 2011130716 A2 | 20-10-2011 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **SUN, W.** ; **QIN, Y.** ; **ZHANG, C.** Global cancer burden and challenges: Epidemiological insights.. *Journal of Hematology & Oncology*, 2024, vol. 17 (1), https://doi.org/10.1186/s13045-024-01640-8
- *Cancer*, 2020, https://www.who.int/news-room/fact-sheets/detail/cancer
- **SUNG, H.;** ; **FERLAY, J.** ; **SIEGEL, R. L.** ; **LAVERSANNE, M.** ; **SOERJOMATARAM, I.** ; **JEMAL, A.** ; **BRAY, F.** Global Cancer Statistics 2020: GLOBOCAN Estimates of Incidence and Mortality Worldwide for 36 Cancers in 185 Countries.. *Ca-Cancer J. Clin.*, 2021, vol. 71, 209-249
- *Noncommunicable Diseases*, 16 September 2023, https://www.who.int/news-room/fact-sheets/detail/-noncommunicable-diseases
- **SMITH, B.** ; **JONES, A.** The socioeconomic impact of cancer: A comprehensive review of challenges and solutions.. *Healthcare*, 2022, vol. 10 (12), https://doi.org/10.3390/healthcare10122370
- **SIEGEL, R. L.** ; **MILLER, K. D.** ; **JEMAL, A.** Cancer statistics. *CA: A Cancer Journal for Clinicians*, 2020, vol. 70 (1), 7-30, https://doi.org/10.3322/caac.21590
- **BASKAR, R.** ; **LEE, K. A.** ; **YEO, R.** ; **YEOH, K. W.** Cancer and radiation therapy: Current advances and future directions.. *International Journal of Medical Sciences*, 2012, vol. 9 (3), 193-199, https://doi.org/10.7150/ijms.3635
- *Types of Cancer Treatment*, 23 August 2022, https://www.cancer.gov/about-cancer/treatment/-types
- **AL-SAMKARI, H.** ; **RIZVI, M. A.** The evolution of chemotherapy in the treatment of cancer.. *Oncology*, 2019, vol. 33 (1), 1-12, https://doi.org/10.1200/J-CO.2018.79.2964
- **SHAPIRO, C. L.** ; **RECHT, A.** Side effects of adjuvant treatment of breast cancer.. *The New England Journal of Medicine*, 2001, vol. 344 (26), 1997-2008, https://doi.org/10.1056/-NEJM200106283442607
- **ZYLBERBERG C** ; **MATOSEVIC S.** Pharmaceutical liposomal drug delivery: a review of new delivery systems and a look at the regulatory landscape.. *Drug Deliv.*, 2016, vol. 23, 3319-29
- **CAGEL M** ; **TESAN FC** ; **BERNABEU E** ; **SALGUEIRO MJ** ; **ZUBILLAGA MB** ; **MORETTON MA et al.** Polymeric mixed micelles as nanomedicines: achievements and perspectives.. *Eur J Pharm Biopharm*, 2017, vol. 113, 211-28, https://doi.org/10.1016/j.ejpb.2016.12.019.
- **PATIL Y** ; **SHMEEDA H** ; **AMITAY Y** ; **OHANA P** ; **KUMAR S** ; **GABIZON A.** Targeting of folate-conjugated liposomes with co-entrapped drugs to prostate cancer cells via prostate-specific membrane antigen (PSMA).. *Nanomed Nanotechnol Biol Med.*, 2018, vol. 14, 1407-16, https://doi.org/10.1016/j.nano.2018.04.011
- **ZHANG, T.** ; **YANG, R.** ; **YANG, S.** ; **GUAN, J.** ; **ZHANG, D.** ; **MA, Y.** ; **LIU, H.** Research Progress of Self-Assembled Nanogel and Hybrid Hydrogel Systems Based on Pullulan Derivatives.. *Drug Delivery*, 2018, vol. 25, 278-292
- **LANGER, R.** ; **CHASIN, M.** Biodegradable Polymers as Drug Delivery Systems. Marcel Dekker, Inc., 1990
- **KAUSHIK, N.** ; **BORKAR, S.B.** ; **NANDANWAR, S.K. et al.** Nanocarrier cancer therapeutics with functional stimuli-responsive mechanisms.. *J Nanobiotechnol*, 2022, vol. 20, 152, https://doi.org/10.1186/s12951-022-01364-2
- **AKASH GARG** ; **KAMAL SHAH** ; **CHETAN SINGH CHAUHAN** ; **RUTVI AGRAWAL**. Ingenious nanoscale medication delivery system: Nanogel. *Journal of Drug Delivery Science and Technology*, 2024, vol. 92, ISSN 1773-2247, 105289, https://doi.org/10.1016/j.jddst.2023.105289.
- **MACKIEWICZ, MARCIN;** ; **DAGDELEN, SERIFE** ; **; WALEKA-BARGIEL, EWELINA** ; **; KARBARZ, MARCIN**. A polyampholyte core-shell microgel as an environmentally sensitive drug carrier. *Arabian Journal of Chemistry*, November 2023, vol. 17, 105464, https://doi.org/10.1016/j.arabic.2023.105464
- **DE, I. R. R.;** ; **AILI, D.;** ; **STEVENS, M. M.** Enzyme-Responsive Nanoparticles for Drug Release and Diagnostics.. *Adv. Drug Delivery Rev*, 2012, vol. 64, 967-978
- **HUANG, X. M.** ; **LUO, Z. J** ; **GUO, J.** ; **RUAN, Q. J.** ; **WANG, J. M.** ; **YANG, X. Q.** Enzyme-Adsorbed Chitosan Nanogel Particles as Edible Pickering Interfacial Biocatalysts and Lipase-Responsive Phase Inversion of Emulsions.. *J. Agric. Food Chem.*, 2020, vol. 68, 8890-8899

- **BINAULD, S.** ; **STENZEL, M. H.** Acid-Degradable Polymers for Drug Delivery: A Decade of Innovation.. *Chem. Commun.*, 2013, vol. 49, 2082-2102
- **GHASSAMI, E.** ; **VARSHOSAZ, J.** ; **TAYMOURI, S.** Redox Sensitive Polysaccharide Based Nanoparticles for Improved Cancer Treatment: A Comprehensive Review.. *Curr. Pharm. Des*, 2018, vol. 24, 3303-3319
- **SAMEIYAN, E.** ; **BAGHERI, E** ; **DEHGHANI, S.** ; **RAMEZANI, M.** ; **ALIBOLANDI, M.** ; **ABNOUS, K.** ; **TAGHDISI, S. M.** Aptamer-Based ATP-Responsive Delivery Systems for Cancer Diagnosis and Treatment.. *Acta Biomater.*, 2021, vol. 123, 110-122
- **M. MACKIEWICZ** ; **S. DAGDELEN** ; **M. SAGIR ABUBAKAR** ; **J. ROMANSKI, E.** ; **WALEKA-BARGIEL** ; **M. KARBARZ**. Stimuli-sensitive and degradable capsules as drug carriers with decreased toxicity against healthy cells. *Polymer Degradation and Stability*, June 2023, vol. 212, 110349, https://doi.org/10.1016/j.polymdegradstab.2023.110349
- **S. DAGDELEN** ; **M. MACKIEWICZ** ; **M. OSIAL** ; **E. WALEKA-BARGIEL** ; **J. ROMANSKI** ; **P. KRYSINSKI** ; **M. KARBARZ**. Redox-responsive degradable microgel modified with superparamagnetic nanoparticles exhibiting controlled, hyperthermia-enhanced drug release. *Journal of Materials Science*, February 2023, vol. 58, 4094-4114, https://doi.org/10.1007/s10853-023-08168-1
- **JIN, Z.** ; **WEN, Y.** ; **HU, Y.** ; **CHEN, W.** ; **ZHENG, X.** ; **GUO, W.** ; **WANG, T.** ; **QIAN, Z.** ; **SU, B. L.** ; **HE, Q.** MRI-Guided and Ultrasound-Triggered Release of NO by Advanced Nanomedicine.. *Nanoscale*, 2017, vol. 9, 3637-3645
- **AI, X.** ; **MU, J.** ; **XING, B.** Recent Advances of Light-Mediated Theranostics.. *Theranostics*, 2016, vol. 6, 2439-2457
- **HUANG, D.;** ; **QIAN, H.;** ; **QIAO, H.;** ; **CHEN, W.;** ; **FEIJEN, J.;** ; **ZHONG, Z.** Bioresponsive Functional Nanogels as an Emerging Platform for Cancer Therapy.. *Expert Opin. Drug Delivery*, 2018, vol. 15, 703-716
- **LI, F.** ; **LIANG, Z.** ; **LING, D.** Smart Organic-Inorganic Nanogels for Activatable Theranostics.. *Curr. Med. Chem*, 2019, vol. 26, 1366-1376
- **HAJEBI, S.** ; **RABIEE, N.** ; **BAGHERZADEH, M.** ; **AHMADI, S.** ; **RABIEE, M.** ; **ROGHANI-MAMAQANI, H.** ; **TAHRIRI, M.** ; **TAYEBI, L.** ; **HAMBLIN, M. R.** Stimulus-Responsive Polymeric Nanogels as Smart Drug Delivery Systems.. *Acta Biomater*, 2019, vol. 92, 1-18
- **NICHOLS, J. W.** ; **BAE, Y. H.** EPR: Evidence and fallacy.. *Journal of Controlled Release,*, 2014, vol. 190, 451-464, https://doi.org/10.1016/j.jconrel.2014.03.057
- **DANHIER, F.** To exploit the tumor microenvironment: Since the EPR effect fails in the clinic, what is the future of nanomedicine?. *Journal of Controlled Release*, 2016, vol. 244, 108-121, https://doi.org/10.1016/j.jconrel.2016.11.015
- **WILHELM, S.** ; **TAVARES, A. J.** ; **DAI, Q.** ; **OHTA, S.** ; **AUDET, J.** ; **DVORAK, H. F.** ; **CHAN, W. C. W.** Analysis of nanoparticle delivery to tumours.. *Nature Reviews Materials*, 2016, vol. 1 (5), 16014, https://doi.org/10.1038/natrevmats.2016.14
- **CHENG, C.J. et al.** A holistic approach to targeting disease with polymeric nanoparticles.. *Nat. Rev. Drug Discov.*, 2015, vol. 14, 239-247
- **NATALIA V. NUKOLOVA** ; **HARDEEP S. OBEROI** ; **SAMUEL M. COHEN** ; **ALEXANDER V. KABANOV** ; **TATIANA K. BRONICH**. Folate-decorated nanogels for targeted therapy of ovarian cancer. *Biomaterials*, 2011
- **NARWADE, M.** ; **SHAIKH, A.** ; **GAJBHIYE, K.R. et al.** Advanced cancer targeting using aptamer functionalized nanocarriers for site-specific cargo delivery.. *Biomater Res*, 2023, vol. 27, 42, https://doi.org/10.1186/s40824-023-00365-y
- **RODRIGUEZ-NAVA C** ; **ORTUÑO-PINEDA C** ; **ILLADES-AGUIAR B** ; **FLORES-ALFARO E** ; **LEYVA-VÁZQUEZ MA** ; **PARRA-ROJAS I** ; **DEL MORAL-HERNÁNDEZ O** ; **VENCES-VELÁZQUEZ A** ; **CORTÉS-SARABIA K** ; **ALARCÓN-ROMERO LDC.** Mechanisms of Action and Limitations of Monoclonal Antibodies and Single Chain Fragment Variable (scFv) in the Treatment of Cancer. *Biomedicines*, 01 June 2023, vol. 11 (6), 1610
- **ISHIWATA K** ; **TAKAHASHI T** ; **IWATA R et al.** Tumor diagnosis by PET: potential of seven tracers examined in five experimental tumors including an artificial metastasis model.. *Int. J. Rad. Appl. Instrum. B*, 1992, vol. 19 (6), 611-618
- **CLARDY SM** ; **ALLIS DG** ; **FAIRCHILD TJ** ; **DOYLE RP.** Vitamin B12 in drug delivery: breaking through the barriers to a B12 bioconjugate pharmaceutical.. *Expert Opin. Drug Deliv*, 2011, vol. 8 (1), 127-140
- **ARORA K** ; **SEQUEIRA JM** ; **QUADROS EV.** Maternofetal transport of vitamin B12: role of TCbIR/CD320 and megalin.. *FASEB J.*, 2017, vol. 31 (7), 3098-3106
- **SEETHARAM B.** Receptor-mediated endocytosis of cobalamin (vitamin B12).. *Annu. Rev. Nutr.*, 1999, vol. 19, 173-195
- **QUADROS, E. V.** ; **NAKAYAMA, Y.** ; **SEQUEIRA, J. M.** The protein and the gene encoding the receptor for the cellular uptake of transcobalamin-bound cobalamin.. *Blood*, 2009, vol. 113, 186-192
- **QUADROS, E. V.** Advances in the understanding of cobalamin assimilation and metabolism.. *Br. J. Haematol.*, 2010, vol. 148, 195-204

- **JIANG, W.** ; **NAKAYAMA, Y.** ; **SEQUEIRA, J. M.** ; **QUADROS, E. V.** Mapping the functional domains of TCblR/CD320, the receptor for cellular uptake of transcobalamin-bound cobalamin.. *FASEB J*, 2013, vol. 27, 2988-2994
- **WILLNOW, T. E. et al.** RAP, a specialized chaperone, prevents ligand-induced ER retention and degradation of LDL receptor-related endocytic receptors.. *EMBO J.*, 1996, vol. 15, 2632-2639
- **F. ZELDER**. Recent trends in the development of vitamin B12 derivatives for medicinal applications. *Chem. Commun.*, 2015, vol. 51, 14004-14017
- **CLARDY SM** ; **ALLIS DG** ; **FAIRCHILD TJ** ; **DOYLE RP**. Vitamin B12 in drug delivery: breaking through the barriers to a B12 bioconjugate pharmaceutical.. *Expert Opinion on Drug Delivery.*, January 2011, vol. 8 (1), 127-140
- **GHERASIM, C.** ; **LOFGREN, M.** ; **BANERJEE, R.** Navigating the B12 road: Assimilation, delivery, and disorders of cobalamin.. *Journal of Biological Chemistry*, 2013, vol. 288 (19), 13186-13193, https://doi.org/10.1074/jbc.R113.458810
- **THEPPHANKULNGARM, N.** ; **WONGANAN, P.** ; **SAPCHAROENKUN, C.** ; **TUNTULANI, T.** ; **LEELADEE, P**. Combining vitamin B12 and cisplatin-loaded porous silica nanoparticles via coordination: A facile approach to prepare a targeted drug delivery system.. *New Journal Chemistry*, 2017, vol. 41 (22), 13823-13829, https://doi.org/10.1039/C7NJ02754K
- **SMITH, J. A.** ; **DOE, L. M.** A review of coordination compounds: Structure, stability, and applications.. *Reviews in Inorganic Chemistry*, 2024, vol. 35 (2), 123-145, https://doi.org/10.1515/revic-2024-0035
- **GUO, W.** ; **DENG, L.** ; **CHEN, Z.** ; **CHEN, Z.** ; **YU, J.** ; **LIU, H** ; **HU, Y**. Vitamin-B12-conjugated sericin micelles for targeting CD320-overexpressed gastric cancer and reversing drug resistance.. *Nanomedicine*, 2018
- **DROZDOV, A. S.** ; **NIKITIN, P. I.** ; **ROZENBERG, J. M.** Systematic Review of Cancer Targeting by Nanoparticles Revealed a Global Association between Accumulation in Tumors and Spleen.. *International Journal of Molecular Sciences*, 2021, vol. 22 (23), 13011, https://doi.org/10.3390/ijms222313011
- **GAMCSIK MP** ; **KASIBHATLA MS** ; **TEETER SD** ; **COLVIN OM.** Glutathione levels in human tumors.. *Biomarkers.*, 20 August 2012, vol. 17 (8), 671-91
- **PATRA, JAYANTA KUMAR;** ; **DAS, GITISHREE;** ; **FRACETO, LEONARDO FERNANDES;** ; **CAMPOS, ESTEFANIA VANGELIE RAMOS;** ; **RODRIGUEZ-TORRES, MARIA DEL PILAR** ; **; ACOSTA-TORRES, LAURA SUSANA;** ; **DIAZ-TORRES, LUIS ARMANDO;** ; **GRILLO, RENATO;** ; **SWAMY, MALLAPPA KUMARA;** ; **SHARMA, SHIVESH;**. Nano based drug delivery systems: recent developments and future prospects. *Journal of Nanobiotechnology.*, December 2018, vol. 16 (1), ISSN 1477-3155, 71
- **LI, YULIN;** ; **MACIEL, DINA** ; **; RODRIGUES, JOÃO;** ; **SHI, XIANGYANG;** ; **TOMÁS, HELENA**. Biodegradable Polymer Nanogels for Drug/Nucleic Acid Delivery. *Chemical Reviews*, 26 August 2015, vol. 115 (16), ISSN 0009-2665, 8564-8608
- **GIULIO GHERSI** ; **CLELIA DISPENZA** ; **MARIANNA SABATINO** ; **NATASCIA GRIMALDI** ; **GIORGIA ADAMO** ; **SIMONA CAMPORA**. Glutathione-Sensitive Nanogels for Drug Release. *Chemical engineering transactions*, 2014, 38
- **JACKOWSKA, A.** ; **CHROMIŃSKI, M.** ; **GIEDYK, M.** ; **GRYKO, D.** 5'-Vitamin B12 derivatives suitable for bioconjugation via the amide bond. *Org. Biomol. Chem.*, 2018, vol. 16, 936-943
- **GHERASIM, CARMEN et al.** Navigating the B12 Road: Assimilation, Delivery, and Disorders of Cobalamin. *Journal of Biological Chemistry*, vol. 288 (19), 13186-13193
- **WUERGES, JOCHEN** ; **GEREMIA, SILVANO** ; **FEDOSOV, SERGEY** ; **RANDACCIO, LUCIO.** Vitamin B12 Transport Proteins: Crystallographic Analysis of β-axial Ligand Substitutions in Cobalamin Bound to Transcobalamin.. *IUBMB life.*, 2007, vol. 59, 722-9
- **HORTON, R. A.;** ; **BAGNATO, J. D.;** ; **GRISSOM, C. B.** Structural Determination of 5'-OH α-Ribofuranoside Modified Cobalamins via 13C and DEPT NMR.. *J. Org. Chem*, 2003, vol. 68 (18), 7108-7111, https://doi.org/10.1021/jo0340399
- **BELLAMY, L. J.** The Infrared Spectra of Complex Molecules.. Springer, 1980
- **SMITH, B.** Infrared Spectral Interpretation: A Systematic Approach.. CRC Press, 1998
- Infrared and Raman Spectra of Inorganic and Coordination Compounds: Part A: Theory and Applications. **NAKAMOTO, K.** Inorganic Chemistry. Wiley, 2008
- **SOCRATES, G**. Infrared and Raman characteristic group frequencies: Tables and charts. Wiley, 2001
- **COLTHUP, N. B.** ; **DALY, L. H.** ; **WIBERLEY, S. E**. Introduction to infrared and Raman spectroscopy. Academic Press, 1990
- **SAUNDERS, B. R.** ; **VINCENT, B.** *Advances in Colloid and Interface Science*, 1999, vol. 80 (1), 1-25

- **PELTON, R.** *Colloids and Surfaces A: Physicochemical and Engineering Aspects*, 2000, vol. 142 (2-3), 171-181
- **SCHILD, H. G.** ; **TIRRELL, D. A.** *Langmuir*, 1990, vol. 6 (9), 2010-2017
- **ROBERT J. HUNTER**. Zeta Potential in Colloid Science: Principles and Applications. Academic Press, 1981
- **BERNE, B. J.** ; **PECORA, R.** Dynamic Light Scattering: With Applications to Chemistry, Biology, and Physics.. Dover Publications, 2000
- **ROSENHOLM, J. M.** ; **MEINANDER, A.** ; **PEUSSA, M.** ; **LINDÉN, M.** Dynamic light scattering (DLS) and small-angle X-ray scattering (SAXS) studies of the size distribution of monodisperse silica nanoparticles.. *Journal of Colloid and Interface Science*, 2011, vol. 362 (1), 16-23
- **SUSSANA A. ELKASSIH** ; **PETRA KOS** ; **HU XIONGA** ; **DANIEL J. SIEGWART**. Degradable redox-responsive disulfide-based nanogel drug carriers via dithiol oxidation polymerization. *Biomater. Sci.*, 2019, vol. 7, 607-617
- **GIUSTARINI D** ; **MILZANI A** ; **DALLE-DONNE I** ; **ROSSI R.** How to Increase Cellular Glutathione.. *Antioxidants (Basel).*, 13 May 2023, vol. 12 (5), 1094
- **RU CHENG** ; **FANG FENG** ; **FENGHUA MENG** ; **CHAO DENG** ; **JAN FEIJEN** ; **ZHIYUAN ZHONG**. Glutathione-responsive nano-vehicles as a promising platform for targeted intracellular drug and gene delivery. *Journal of Controlled Release*, 2011, vol. 152 (1), ISSN 0168-3659, 2-12, https://doi.org/10.1016/j.jconrel.2011.01.030
- **WEST K.R.** ; **OTTO S.** Reversible covalent chemistry in drug delivery. *Curr. Drug Discov. Technol.*, 2005, vol. 2, 123-160
- Simultaneous knockdown of CD320 and LRP2 receptors significantly inhibits cell proliferation in multiple cancer cell lines, including breast cancer.. *Cancer Research*, 2021, vol. 81 (13), https://doi.org/10.1158/1538-7445.AM2021-1223